(19) Europäisches Patentamt / European Patent Office / Office européen des brevets

(11) **EP 4 528 744 A1**

(12) **EUROPEAN PATENT APPLICATION**

(43) Date of publication:
26.03.2025 Bulletin 2025/13

(21) Application number: 24199929.1

(22) Date of filing: 12.09.2024

(51) International Patent Classification (IPC):
*G16H 20/17* (2018.01) *G16H 50/50* (2018.01)

(52) Cooperative Patent Classification (CPC):
**G16H 20/17; G16H 50/50**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 15.09.2023 US 202363583095 P
19.08.2024 US 202418808406

(71) Applicant: **MEDTRONIC MINIMED, INC.**
**Northridge, CA 91325-1219 (US)**

(72) Inventors:
• **STONE, Michael P.**
**Northridge, 91325 (US)**
• **MIKHNO, Arthur**
**Northridge, 91325 (US)**
• **ZHONG, Yuxiang**
**Northridge, 91325 (US)**

(74) Representative: **Zimmermann & Partner**
**Patentanwälte mbB**
**Postfach 330 920**
**80069 München (DE)**

(54) **TREND BASED ADJUSTMENTS TO INSULIN DOSAGE**

(57) Disclosed herein are techniques related to making trend based adjustments to insulin dosage. In some embodiments, the techniques may involve obtaining, based on glucose measurements performed on a person, a glucose value and a glucose rate of change (ROC). An initial insulin amount to deliver to the person is calculated based on this glucose value. However, an adjustment value can be obtained in response to a determination that the glucose value exceeds a glucose threshold, and that the glucose ROC exceeds an ROC threshold. The adjustment value represents an adjustment to be applied to the initial insulin amount when the glucose threshold and the ROC threshold are both exceeded. The adjustment value can be predetermined, based on earlier glucose measurements performed on the person. For instance, the adjustment value and the thresholds can be jointly determined through a computer simulation using a person-specific physiological model.

**FIG. 4**

EP 4 528 744 A1

**Description**

CROSS-REFERENCE TO RELATED APPLICATIONS

This application claims the benefit of U.S. Provisional Application No. 63/583,095, filed September 15, 2023, entitled "TREND BASED ADJUSTMENTS TO INSULIN DOSAGE" which is incorporated herein by reference in its entirety.

TECHNICAL FIELD

The present disclosure relates generally to medical devices, in particular, to therapy delivery devices and systems including therapy delivery devices. Certain aspects of the disclosure are directed to techniques for adjusting insulin dosage in conjunction with glucose monitoring.

BACKGROUND

Some medical conditions such as diabetes necessitate monitoring of a patient's corresponding physiological condition (e.g., glucose level) for purposes of determining when to deliver a dose of a therapeutic agent. Delivery can be automated, for example, using a continuous glucose monitoring (CGM) device that controls an insulin infusion pump based on glucose measurements obtained by the CGM device. Therapeutic agents can also be delivered manually, for example, using a syringe to inject a premeasured dose of insulin. Glucose monitoring is traditionally performed on blood samples, e.g., using fingerstick measurements. Blood glucose measurements are increasingly less common over alternative sensing methods such as interstitial glucose sensors, which permit glucose to be measured in a more convenient manner and with less discomfort.

The amount of a dose is sometimes determined based on the patient's physiological condition at or around the time the dose is to be delivered. For example, a number of units of insulin to deliver can be determined based on the patient's glucose level as measured by a blood glucose meter, interstitial glucose sensor, or other glucose sensing means. When the dosage is determined based on sensor measurements, the accuracy of the measurements can significantly affect patient health outcome. In the case of insulin delivery, too much insulin can lead to hypoglycemia, and too little insulin can lead to hyperglycemia. Further, rapid changes in physiological condition can render a measurement obsolete, even if the dose is delivered shortly after the measurement (e.g., within several minutes). As such, determining an appropriate dosage can be challenging in the presence of changing physiological condition.

SUMMARY

Disclosed herein are examples of medical devices and related systems and methods for adjusting the dosage of a medication or therapeutic agent, based on detecting trends in a physiological condition of a person receiving the dose. In certain aspects, the present disclosure relates to adjusting an insulin dose when a person's glucose level and glucose rate each exceed a respective threshold. Aspects of the present disclosure also relate to determining a glucose level threshold and a glucose rate of change threshold which, if exceeded, indicate that a hypoglycemic or hyperglycemic excursion is likely to occur in the absence of making an insulin dose adjustment. In some embodiments, thresholds are determined together with corresponding adjustment factors through running a computer simulation on a physiological model customized for the person receiving the dose. The techniques may be practiced in a variety of ways, such as using a processor-implemented method; a system comprising one or more processors and one or more processor-readable media; and/or one or more (non-transitory) processor-readable media.

In some embodiments, the techniques may involve obtaining, based on glucose measurements performed on a person, a glucose value and a glucose rate of change (ROC) value. An initial insulin amount to deliver to the person is calculated based on the glucose value. The techniques may further involve determining that the glucose value exceeds a glucose threshold, and that the glucose ROC exceeds an ROC threshold. In response to this determination, an adjustment value can be obtained which represents an adjustment to be applied to the initial insulin amount when the glucose threshold and the ROC threshold are exceeded. The initial insulin amount is then adjusted according to the adjustment value. The adjustment value can be determined based on earlier glucose measurements performed on the person. For instance, the earlier glucose measurements may have been used to create a person-specific physiological model for the simulation discussed above.

Further disclosed herein are techniques related to making trend based adjustments to insulin dosage. In some embodiments, the techniques may involve obtaining, based on glucose measurements performed on a person, a glucose value and a glucose rate of change (ROC). An initial insulin amount to deliver to the person is calculated based on this glucose value. However, an adjustment value can be obtained in response to a determination that the glucose value exceeds a glucose threshold, and that the glucose ROC exceeds an ROC threshold. The adjustment value represents an adjustment to be applied to the initial insulin amount when the glucose threshold and the ROC threshold are both exceeded. The adjustment value can be predetermined, based on earlier glucose measurements performed on the person. For instance, the adjustment value and the thresholds can be jointly determined through a computer simulation using a person-specific physiological model

This summary is neither intended to identify key or essential features of the claimed subject matter, nor is it intended to be used in isolation to determine the scope of the claimed subject matter. The subject matter should be understood by reference to appropriate portions of the entire specification of this disclosure, any or all drawings, and each claim. The foregoing, together with other features and examples, will be described in more detail below in the following specification, claims, and accompanying drawings.

BRIEF DESCRIPTION OF THE DRAWINGS

The above and other aspects and features of the disclosure will become more apparent in view of the following detailed description when taken in conjunction with the accompanying drawings wherein like reference numerals identify like elements.

FIG. 1 shows an example therapy system, in accordance with aspects of the present disclosure.
FIG. 2 shows an example of a glucose model usable for performing a simulation, in accordance with aspects of the present disclosure.
FIG. 3 shows an example of a memory subsystem storing adjustment-related information, in accordance with aspects of the present disclosure.
FIG. 4 shows an example of adjustment parameters, in accordance with aspects of the present disclosure.
FIGS. 5A and 5B show examples of glucose excursion rate as a function of glucose rate of change.
FIG. 6 shows the relationship between time-in-range and time-below-range in a sample population when using the same set of adjustment parameters for the entire population.
FIG. 7 includes a comparison of time-in-range with and without dosage adjustments.
FIG. 8 shows example adjustment parameter values used to determine an adjustment parameter set, in accordance with aspects of the present disclosure.
FIGS. 9A and 9B show an example of the results of an insulin adjustment.
FIGS. 10A and 10B show another example of the results of an insulin adjustment.
FIG. 11 shows an example process for adjusting dosage, in accordance with aspects of the present disclosure.
FIG. 12 shows an example process for determining adjustment parameters, in accordance with aspects of the present disclosure.
FIG. 13 shows an example process for overriding an adjustment, in accordance with aspects of the present disclosure.
FIG. 14 shows an example therapy delivery system, in accordance with aspects of the present disclosure.
FIG. 15 shows an example insulin delivery device usable for implementing one or more embodiments described herein.

DETAILED DESCRIPTION

Disclosed herein are examples of medical devices and related systems and methods for adjusting insulin dosage based on detecting trends in a physiological condition of a person receiving the dose. Aspects of the present disclosure are directed to providing a person-specific approach to adjusting insulin dosage when glucose levels are rapidly changing. Aspects of the present disclosure also relate to determining adjustment parameters and applying those adjustments parameters in a therapy context. In some instances, adjustment parameters may be ignored for purposes of setting the amount of a dose.

Although the present disclosure is described primarily with respect to insulin delivery systems, the scope of the present disclosure is not limited to insulin delivery systems. Rather, the present disclosure applies to and can be implemented with other therapy systems as well. For example, in some embodiments, the techniques of the present disclosure may be practiced in relation to pain therapy.

Discussions utilizing terms such as, for example, "processing," "computing," "calculating," "determining," "establishing," "analyzing," "checking," or the like, may refer to operations and/or processes performed by a computer, a computing platform, a computing system, or other electronic device equipped with one or more hardware-implemented processing units (e.g., a microprocessor).

Unless explicitly stated, the methods described herein are not constrained to a particular order or sequence. Additionally, some of the described methods or elements thereof can occur or be performed simultaneously or concurrently. In some instances, the described methods involve operations and/or processes performed by different electronic devices that are communicatively intercoupled to form a therapy delivery system. Unless stated otherwise or contraindicated by context, operations or processes performed by one device (e.g., a server computer) may also be performed, in whole or in part, by another device in the therapy delivery system (e.g., a computing device in communication with the server).

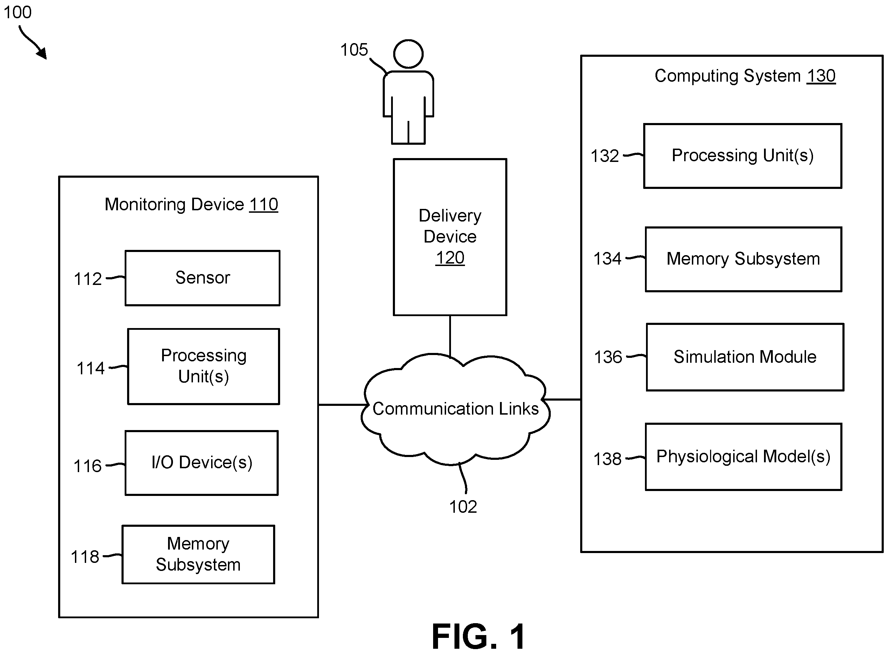

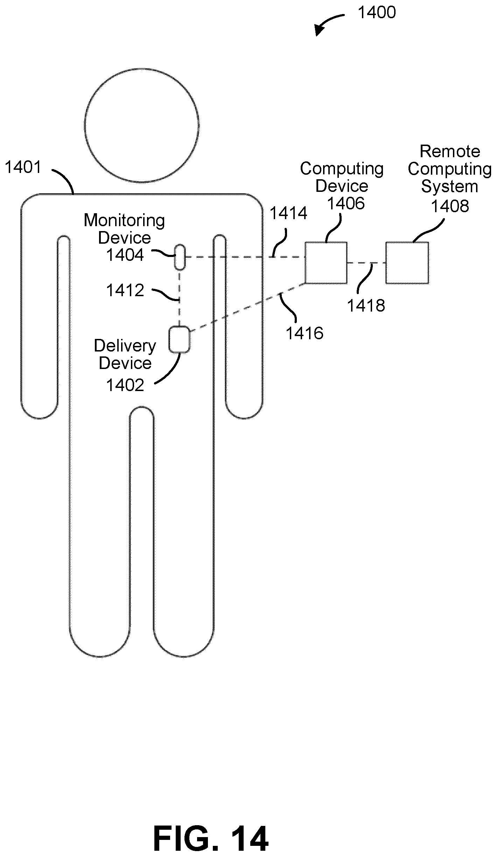

**FIG. 1** shows an example of a therapy delivery system 100 including a monitoring device 110, a delivery device 120, and a computing system 130, which are communicatively coupled through a set of communication links 102. The communication links 102 can include wired and/or wireless connections. In some instances, the communication links 102 may form one or more communication networks. For example, communication links 102 may be part of the Internet, a cellular network, a Wi-Fi network, a local area network, and/or the like. FIG. 1 is provided to illustrate one way in which functionality related to dosage determination may be distributed across different devices in a therapy delivery system. FIG. 14 (described below) shows another example of a therapy delivery system with similar components but more detail on how individual components may be communicatively coupled.

Monitoring device 110 is configured to obtain measurements from a sensor 112 and to, based on the measurements, determine a dose of therapeutic agent (e.g., insulin) to be delivered by the delivery device 120. The sensor 112 is configured to measure a physiological condition of a person 105. As shown in FIG. 1, the sensor 112 can be part of the monitoring device 110. For example, the monitoring device 110 can be a CGM device, and the sensor 112 can be an interstitial glucose sensor integrated into the CGM device. As such, the monitoring device 110 and the sensor 112 may be a single unit worn on or affixed to the body of the person 105. However, the sensor 112 may be remote from the monitoring device 110 in some instances, such as when the sensor 112 is implanted inside the person's body.

Monitoring device 110 is equipped with one or more processing units 114 that process the measurements from the sensor 112 to determine the timing of a dose and the amount of the dose. The sensor 112 can send the measurements to the monitoring device 110 as numeric values characterizing the physiological condition, e.g., digitally encoded glucose values. Alternatively, the sensor 112 can output the measurements in the form of one or more electrical signals produced under the influence of the physiological condition. Accordingly, the processing unit(s) 114 may be configured to convert the values of the one of more electrical signals (voltage, current, impedance, etc.) into corresponding measurement values.

The monitoring device 110 can track the sensor measurements to detect when a trigger condition for dosing has been met, thereby determining the timing of a dose. In diabetes management, insulin doses are generally categorized as basal dosages or bolus dosages. Basal doses are delivered less frequently (e.g., once or twice a day) and are designed to keep glucose levels steady throughout the day, often with slow-acting insulin. The amount of a basal dose can vary between patients but is typically the same from one dose to the next. By contrast, bolus doses are designed to counteract the effects of carbohydrates consumed during meals. Bolus doses are usually done right before a meal but can also be delivered after meals, often with fast-acting insulin. Aspects and embodiments of the present disclosure can be practiced with one or more types of insulin (e.g., fast-acting insulin, intermediate-acting insulin, and/or slow-acting insulin). For example, the delivery device 120 may be configured to use fast-acting insulin for both basal dosages and bolus dosages.

Based on the discussion above, it will be understood that a trigger condition for a basal dose can differ from a trigger condition for a bolus dose. For example, the monitoring device 110 may be programmed or otherwise configured to initiate a basal dose at a certain time of day, and to initiate a bolus dose in response to user input indicating that a meal is about to be consumed. As such, the monitoring device 110 may include one or more input/output (I/O) devices 116 to interact with a user (e.g., the person 105). Examples of I/O devices 116 include display monitors, audio speakers, touchscreens, haptic feedback devices (e.g., to present vibration alerts), keyboards or keypads, mice or trackpads, and/or the like. The I/O device(s) 116 may be used to inform the user about their glucose level and the quantity of insulin delivered. I/O device(s) 116 may also be used to provide the monitoring device 110 with information about the user. For example, user input to the I/O device(s) 116 may include age, height, weight, an insulin sensitivity factor (ISF), an insulin-to-carbohydrate ratio (ICR), and/or other information about the person 105 that the processing unit(s) 114 can use to compute the amount of a dose.

Monitoring device 110 further includes a memory subsystem 118. The memory subsystem 118 can be formed using volatile and/or non-volatile memory devices such as random access memory, read-only memory, flash memory, a solid-state drive, a hard disk drive, etc. In some implementations, the memory subsystem 118 stores instructions executable by the processing unit(s) 114 to cause the monitoring device 110 to calculate a dosage amount and communicate the calculated dosage to the delivery device 120. For example, the memory subsystem 118 may store program code implementing a bolus calculation algorithm according to the formula:

$$B = CARB * \frac{1}{ICR} + (BG - Target) * \frac{1}{ISF} - IOB \qquad (1)$$

where $B$ is a bolus amount to be delivered, $CARB$ is the amount of carbohydrates expected to be consumed (or recently consumed), $ICR$ is the person's insulin-to-carbohydrate ratio, $ISF$ is the person's insulin sensitivity factor, $Target$ is a target glucose level (e.g., a value between 70-180 milligrams per deciliter (mg/dL)), and $BG$ is the person's blood glucose level.

*ICR* represents the amount of carbohydrates (e.g., in grams) that one unit of insulin is expected to cover. *ISF* represents the amount of blood glucose (e.g., in mg/dL) that one unit of insulin is expected to lower. *IOB* stands for insulin on board and represents the amount of residual insulin leftover in the person's body from an earlier dose.

As mentioned above, the sensor 112 can be an interstitial glucose sensor. Interstitial glucose tends to lag behind blood glucose, e.g., by several minutes or more. This is because glucose takes time to diffuse from the bloodstream into the interstitial fluid between the skin and the blood vessels. The monitoring device 110 may be configured to estimate BG from sensor glucose (SG) taking this lag into account when the measurements are from an interstitial glucose sensor. Alternatively, to reduce the computational burden on the processing unit(s) 114, the monitoring device 110 may substitute SG for BG when computing the bolus amount according to Equation 1. When using SG instead of BG, the monitoring device 110 can compensate for sensor lag by adjusting the dosage amount when the dose is delivered under conditions in which the lag is expected to have a disproportionately negative effect on the efficacy of the calculated amount. Such adverse conditions correspond to times when the sensor glucose values are changing rapidly, e.g., increasing or decreasing with a rate of change (ROC) that exceeds a certain magnitude in either direction.

As described in detail below, adjustments can be applied using person-specific adjustment parameters, including an adjustment factor (e.g., a scaling factor) that modifies the dosage amount. Aspects of the present disclosure are directed to methods for determining adjustment parameters through simulating a person's glucose levels under different dosage scenarios, including different rates of change. In addition to the adjustment factor, the adjustment parameters can also include threshold values associated with conditions under which an adjustment is to be applied. For instance, a threshold ROC may be defined to trigger an adjustment when the threshold ROC is exceeded. The adjustment factor can be determined together with the threshold values, based on the results of the simulation. Further, the simulation can be performed in advance, prior to obtaining measurements that lead to the adjustment being applied. In some implementations, the simulation is performed by the computing system 130, which may have access to more computing resources (processing power, memory, bandwidth, etc.) than the monitoring device 110. In this manner, the adjustment can be applied without imposing a significant computational burden on the monitoring device 110 at dosing time.

Irrespective of which component in the system 100 determines the adjustment factor, the monitoring device 110 may store the adjustment factor in the memory subsystem 118 for subsequent use with respect to the person 105. The monitoring device 110 may store the adjustment factor together with the aforementioned threshold values. The threshold values and the adjustment factor may collectively form adjustment parameters that define one or more rules evaluated by the monitoring device 110 to determine when to apply an adjustment and the extent to which a dose is adjusted. Such rule-based adjustments are typically performed with respect to bolus doses but can also be applied to basal dosages when the conditions in the rule(s) are satisfied.

Delivery device 120 is configured to deliver a dose of therapeutic agent under the control of the monitoring device 110. For instance, the delivery device 120 can be an insulin pump that delivers insulin from a reservoir into the body of the person 105 subcutaneously or percutaneously, using a needle, cannula, or other instrument. As such, the delivery device 120 may be communicatively coupled to the monitoring device 110 to receive dosing information indicating when to deliver a dose and the amount of the dose. In some implementations, the monitoring device 110 may schedule the dose ahead of time by sending the delivery device 120 a timestamp indicating the delivery time. Alternatively or additionally, the monitoring device 110 may output a control signal that activates the delivery device 120 at the time of the dose.

To indicate the dosage amount (e.g., the number of units of insulin), the monitoring device 110 can communicate an adjusted amount corresponding to an initially calculated amount with the adjustment factor applied. Thus, the delivery device 120 may receive the final value of the dose. Another way the monitoring device 110 can indicate the dosage amount is to communicate both the initially calculated amount and the adjustment factor so that the delivery device 120 is able to calculate the adjusted amount.

In some instances, the delivery device 120 is a manually operated device. For example, the delivery device 120 can be a reusable injection pen equipped with a communication interface (e.g., a wireless transceiver) for communicating with the monitoring device 110 and/or another component in the system 100. When operated by a user (e.g., the user of the monitoring device 110 and/or the person 105 receiving the dose), the injection pen may communicate the amount of medication delivered to help the monitoring device 110 keep track of doses.

Computing system 130 includes one or more processing units 132 and a memory subsystem 134. In some instances, the computing system 130 may be a server computer or a cloud computing network with multiple servers. The processing unit(s) 114 and the memory subsystem 134 may be configured similarly to the processing unit(s) 114 and the memory subsystem 118 of the monitor device. However, as mentioned above, the computing system 130 may have access to more computing resources. For instance, the computing system 130 may have a greater number of processors, faster processors, and/or a larger storage capacity. Further, the computing system 130 may be configured to perform certain operations in parallel. For example, the simulation for determining the adjustment parameters can be performed across a set of cloud servers, with each cloud server running the simulation across a different search space. Thus, a first cloud server may determine an optimal adjustment factor and corresponding threshold values for a first set of scenarios, a second cloud server may determine an optimal adjustment factor and corresponding threshold values for a second set of scenarios, and the results may be combined by the first cloud server, the second cloud server, or a third cloud server to make a final determination of the adjustment parameters.

Computing system 130 further includes a simulation module 136 and one or more physiological models 138. The simulation module 136 is configured to perform simulations using the physiological model(s) 138. The physiological model(s) 138 may include at least one glucose model describing the glucose response of the person 105 under different scenarios. The computing system 130 may store models for other people besides the person 105. As such, the computing system 130 may determine individualized adjustment parameters for multiple people (e.g., a group of patients) and communicate these adjustment parameters to their respective monitoring devices.

Simulation module 136 can be implemented in hardware and/or software. For instance, the simulation module 136 can be formed using the processing unit(s) 132. In some implementations, the simulation module 136 includes program code containing instructions that cause the processing unit(s) 132 to execute the simulation and/or perform related operations. Accordingly, the simulation module 136 may correspond to a software program executed as part of configuring the monitoring device 110 to perform dosage adjustments. The adjustment parameters can be updated and communicated to the monitoring device 110 repeatedly during the lifetime of the monitoring device. For example, the computing system 130 may periodically rerun the simulation using new measurements (e.g., on a monthly basis) to determine whether the adjustment parameters can be improved by changing one or more values. If so, the computing system 130 can communicate updated adjustment parameters to reconfigure the monitoring device 110.

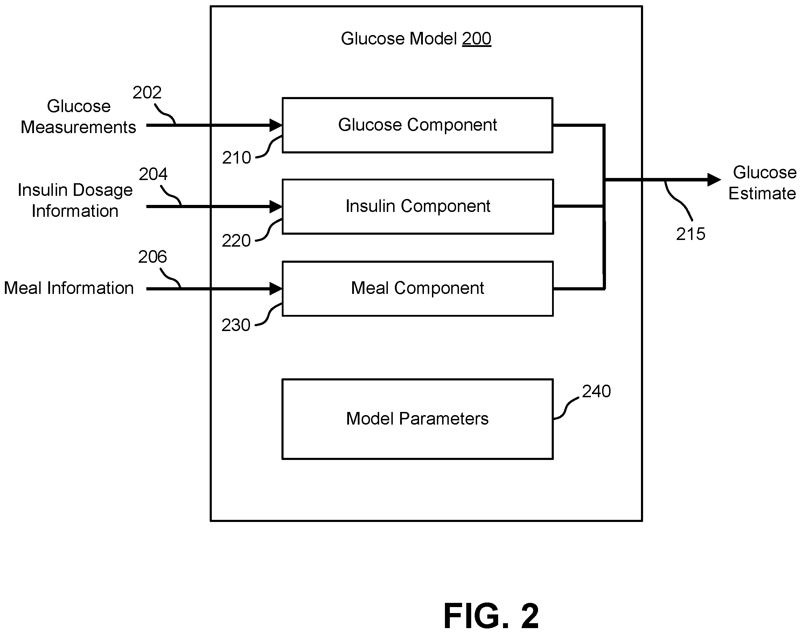

Physiological model(s) 138 can include any number of predictive models adapted to estimate the physiological condition of the person 105. In the case of glucose level, various glucose models exist which differ in modeling approach and model complexity. FIG. 2 (discussed below) shows a simplified representation of an example glucose model. In general, a glucose model is configured to output an estimate of glucose level at a particular point in time or over a specific time period.

Traditional glucose models are mathematical models based on deterministic equations that characterize the glucose-insulin response. A glucose model can be applied to predict what someone's glucose level will be in the near future (e.g., in the next half hour) and to evaluate how their glucose level is expected to change, based on present conditions (e.g., current glucose level) and further based on knowledge of glucose-influencing activities such as meals or exercise. Thus, the input to a glucose model may include, among other things, glucose measurements (e.g., one or more SG values) and insulin dosage information (e.g., the number of units of insulin along with time of delivery). However, the techniques described herein can be applied to models of other physiological conditions. Further, the described techniques are not limited to mathematical models but may also be used with other types of models, which can be non-deterministic (e.g., a statistical/probabilistic model) and/or machine-learning based (e.g., a neural network model).

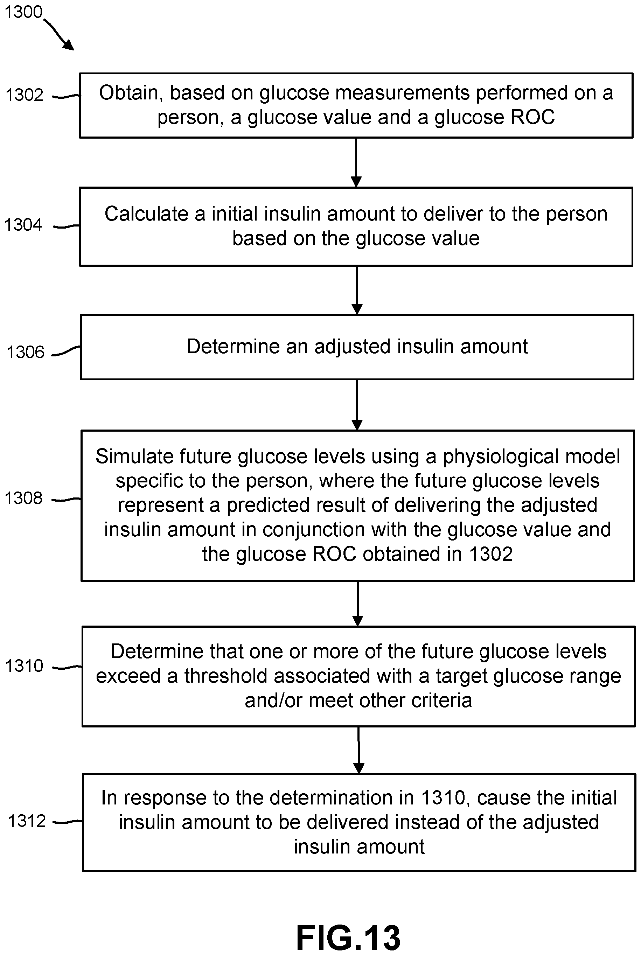

In some embodiments, the simulation using the person-specific model can be performed by another component besides the computing system 130. For example, the computing system 130 may determine adjustment parameters using a comparatively large number of historical measurements performed on the person 105. The historical measurements can include measurements obtained from the sensor 112 and/or other sensors, for example, the sensor 112 in a previous instance of the monitoring device 110. After being configured according to the adjustment parameters, the monitoring device 110 may periodically rerun the simulation using a smaller set of measurements (e.g., only measurements obtained after the initial configuration) and/or over a narrower search space (e.g., within a certain value range of the adjustment parameters). As discussed below in connection with FIG. 13, this rerunning of the simulation can be performed to override an adjustment without necessarily updating the adjustment parameters. Accordingly, the physiological model(s) 138 may be shared across multiple computing systems or devices. For example, the monitoring device 110 may be provided with a generic physiological model, and the computing system 130 may be responsible for customizing the model based on model parameters specific to the person 105. After obtaining the model parameters (e.g., through computation and/or user input), the computing system 130 may communicate the model parameters to the monitoring device 110 for simulation use or other purposes.

**FIG. 2** illustrates an example of a glucose model 200 that can be used to generate a glucose estimate 215 as part of performing a simulation. The glucose model 200 may correspond to a physiological model 138 in FIG. 1. The glucose model 200 includes three components or compartments: a glucose component 210, an insulin component 220, and a meal component 230. Each component of the glucose model 200 has one or more inputs associated with it. The input to the glucose component 210 includes glucose measurements 202, which can be BG and/or SG values from a sensor such as the sensor 112. The input to the insulin component 220 includes insulin dosage information 204, e.g., units of insulin. The input to the meal component includes meal information 206, e.g., number of carbohydrates, as indicated in Equation 1 above.

The glucose component 210 describes glucose dynamics and may be characterized by the following equations:

$$\dot{G}(t) = -p_1 * G(t) - SI * I_p(t) + m_1 * \frac{R_a(t)}{(V_g * BW)} + G_{endo} \qquad (2)$$

$$\dot{G}_{sc}(t) = -h_c * G_{sc}(t) + p_1 * G(t) \qquad (3)$$

Equation 2 describes glucose in the bloodstream as being equal to the sum of glucose produced natively in the body and glucose contributed by a meal, minus glucose lowered via insulin action, and minus glucose diffused from blood plasma into the interstitial fluid. Equation 3 describes subcutaneous (e.g., interstitial) glucose as being equal to the glucose diffused from blood minus the glucose taken up by the surrounding subcutaneous (e.g., fat or muscle) cells. $G$ is the blood glucose level. $p_1$ is the glucose clearance rate independent of insulin. $SI$ is an insulin sensitivity index similar to $ISF$ in Equation 1, but may be expressed in different units, e.g., (dL/minute)/(μU/mL). $I_p$ stands for plasma insulin and is the amount of insulin in the blood (indicated by the insulin component). $m_1$ is the speed of carbohydrate absorption. $R_a$ is the rate of glucose appearance in the blood after a meal (indicated by the meal component). $BW$ is body weight. $V_g$ is the distribution volume of glucose and is proportional to body weight. $G_{endo}$ is the concentration of endogenously produced glucose. $G_{sc}$ stands for subcutaneous glucose, e.g., the concentration of glucose in the interstitial fluid. $h_c$ is the rate of glucose uptake into the subcutaneous tissue.

The insulin component 220 describes insulin dynamics and may be characterized by the following equations:

$$\dot{I}_{sc}(t) = -k_d * I_{sc}(t) + k_d * J(t) \qquad (4)$$

$$\dot{I}_p(t) = k_d * I_{sc}(t) - k_{cl} * I_p(t) \qquad (5)$$

Equation 4 describes subcutaneous insulin as being equal to insulin delivered and subsequently absorbed, minus insulin diffused into the blood. Equation 5 describes insulin in the blood as being equal to the insulin coming from the interstitial fluid, minus insulin cleared through insulin metabolism. $I_{sc}$ stands for subcutaneous insulin, e.g., the concentration of insulin in the interstitial fluid. $k_d$ is a parameter representing the speed of subcutaneous insulin absorption. $J$ represents the amount of insulin delivered as a function of time. $I_p$ is the plasma insulin level. $k_{cl}$ is a parameter representing the speed of plasma insulin absorption.

The meal component 230 describes the glucose contribution of meals and may be characterized by the following equations:

$$\dot{M}(t) = -m_1 * M(t) + k_m * meal(t) \qquad (6)$$

$$\dot{R}_a(t) = m_1 * M(t) - m_1 * R_a(t) \qquad (7)$$

Equation 6 describes the glucose contribution of a meal as being equal to glucose converted from carbohydrates, minus glucose absorbed into the bloodstream from the digestive system. Equation 7 describes the rate of appearance of glucose in the blood ($R_a$ in Equation 2) as being equal to glucose from the digestive system, minus glucose appearing in the blood as a result of the meal. M represents the amount of glucose available due to a meal. $m_1$ is the rate of carbohydrate absorption. $k_m$ is a carbohydrate-to-glucose conversion factor. $meal(t)$ describes carbohydrate intake as a function of time.

From the discussion of Equations 2 to 7 above, it is understood that some of the parameters in the glucose model 200 are person-specific and/or subject to change over time (e.g., body weight or insulin sensitivity index). Accordingly, when applied to the example in FIG. 1, the glucose model 200 may include model parameters 240 that are stored in association with the person 105. The model parameters 240 can be obtained in various ways including, for example, through user input to the monitoring device 110 or the computing system 130. In some instances, one or more model parameters may be computed, e.g., as part of fitting a physiological model to measurements or other data associated with the person 105.

FIG. 2 is provided merely as an example of a physiological model that can be used to simulate different scenarios. Other physiological models can also be used in conjunction with the techniques described herein, including other types of glucose models. Further, as indicated above, the physiological model(s) 138 are not limited to mathematical models. For instance, a neural network model could be trained to infer glucose level based on inputs similar to those used by the glucose model 200. The training of the neural network model may involve using glucose measurements and other data associated with a general population as an initial training data set. Afterwards, the neural network model could be further trained using data associated with the person 105 (e.g., measurements from the sensor 112, meal history, recorded body weight, etc.).

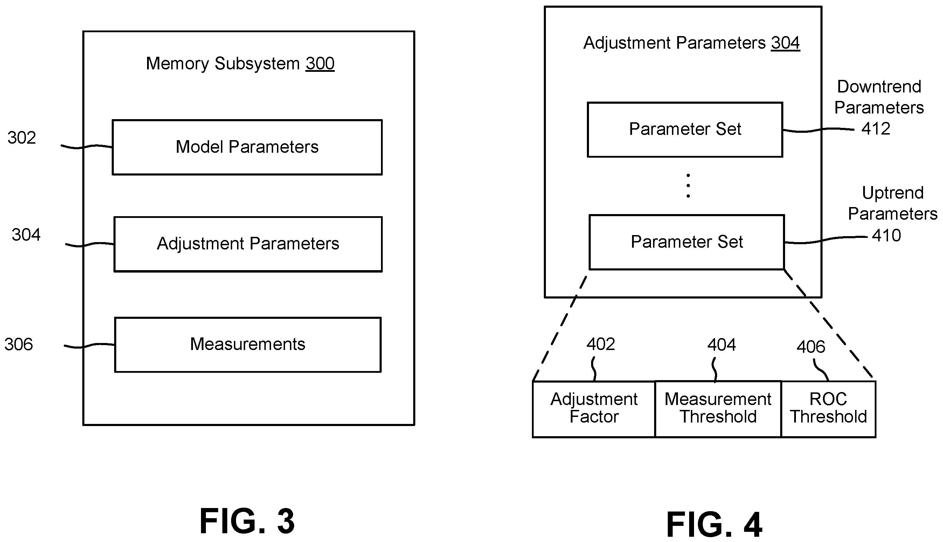

**FIG. 3** shows an example of a memory subsystem 300 storing adjustment-related data. The memory subsystem 300 may correspond to the memory subsystem 118 of the monitoring device 110 and/or the memory subsystem 134 of the computing system 130. As shown in FIG. 3, the memory subsystem 300 can store model parameters 302, adjustment parameters 304, and measurements 306. The contents of the memory subsystem 300 can be stored in a centralized or distributed manner and using any number of data structures. For example, if the memory subsystem 300 is the memory subsystem 118 in FIG. 1, the model parameters 302 and the measurements 306 could be stored external to the processing unit(s) 114, while the adjustment parameters 304 could be stored in a processor-level cache and/or hardware register for faster access by the processing unit(s) 114.

Model parameters 302 can include parameters used by the physiological model(s) 138 to estimate glucose level or the value of another physiological condition. For example, the model parameters 302 may include values for any of the person-specific variables in Equations 2 to 6 above. The model parameters 302 can also include parameters used to calculate an initial dosage (e.g., *ICR* and *ISF* in Equation 1). Accordingly, the model parameters 302 can be accessed in connection with performing a simulation to determine the adjustment parameters 304 and/or in connection with determining the size of a dose.

Adjustment parameters 304 are parameters used to adjust a dosage amount. As discussed earlier, adjustment parameters can include an adjustment factor and threshold values associated with the conditions under which an adjustment will be applied. FIG. 4 shows an example of how the adjustment parameters 304 may be arranged. However, other implementations of adjustment parameters are possible.

Measurements 306 can include measurements performed on the person 105. The measurements 306 can be collected using one or more sensors and over any period of time. For example, measurements for determining the adjustment parameters 304 may be collected over the course of a week or longer. The number of measurements collected depends on sampling frequency. For example, a CGM device may obtain measurements in 1, 5, or 15 minute intervals. The measurements 306 may include interstitial glucose readings (e.g., SG values from an interstitial glucose sensor). In some instances, the measurements 306 may include reference values (e.g., BG values from a blood glucose meter). BG values tend to be collected less frequently, so SG values would typically outnumber BG values when SG and BG are both collected. The measurements 306 can also include measurements collected after the adjustment parameters 304 have been determined, e.g., measurements used to compute a dose according to Equation 1. The number of measurements considered for dosing purposes is typically significantly less than the number of measurements used to determine the adjustment parameters 304.

**FIG. 4** shows an example of adjustment parameters arranged into parameter sets. In FIG. 4, the adjustment parameters 304 include a first parameter set 410 and a second parameter set 412. Each parameter set includes an adjustment factor and corresponding threshold values. The threshold values specify the conditions under which the adjustment factor will be applied to modify a dose. For instance, the first parameter set 410 may include parameters applicable to times when glucose level is trending upwards. As such, the first parameter set 410 may include an adjustment factor 402 and a measurement threshold 404 representing the lowest glucose value at which the adjustment factor 402 will be applied. The first parameter set 410 may further include an ROC threshold 406 representing the lowest glucose rate of change at which the adjustment factor 402 with be applied. Consequently, use of the adjustment factor 402 would be conditioned upon measured glucose (BG or SG) being higher than the measurement threshold 404 and glucose ROC being higher than the ROC threshold 406. The first parameter set 410 could therefore be expressed in terms of the logical rule: if measured glucose > measurement threshold and glucose ROC > ROC threshold, then increase dose according to adjustment factor.

Similarly, the second parameter set 412 may include a separate adjustment factor 402, a corresponding measurement threshold 404, and a corresponding ROC threshold 406, which are applicable to times when glucose level is trending downwards. The measurement threshold 404 of the second parameter set 412 may represent the highest glucose value at which the adjustment factor 402 of the second parameter set 412 will be applied. The ROC threshold 406 of the second parameter set 412 may represent the highest glucose rate of change at which the adjustment factor 402 of the second parameter set 412 with be applied. Consequently, use of the adjustment factor in the second parameter set 412 would be conditioned upon measured glucose being lower than the measurement threshold 404 and glucose ROC being lower than the ROC threshold 406. The second parameter set 412 could therefore be expressed in terms of the logical rule: if measured glucose < measurement threshold and glucose ROC < ROC threshold, then decrease dose according to adjustment factor.

The adjustment factor 402 can be configured to provide an increase or a decrease in a calculated dosage depending on which threshold conditions are satisfied. In the up-trending case, rapidly increasing glucose level (characterized by a positive ROC value) usually warrants an increase in insulin. The adjustment factor 402 of the first parameter set 410 could therefore be a scaling factor multiplied with the calculated dosage, with the scaling factor being a value greater than one. Conversely, in the down-trending case, rapidly decreasing glucose level (characterized by a negative ROC value) usually warrants a decrease in insulin. The adjustment factor 402 of the second parameter set 412 could therefore be a scaling factor having a value less than one. The adjustment factors in the parameter sets 410 and 412 can modify doses to different degrees. For example, the parameter set 410 may provide a 60% increase using a scaling factor of 1.6, whereas the parameter set 412 may provide a 50% decrease using a scaling factor of 0.5.

FIG. 4 is provided as an example of how threshold values relating to the rate of change in a physiological condition can be used to specify the circumstances in which a dosage will be adjusted. In the example of FIG. 4, each parameter set comprises a measurement threshold in combination with an ROC threshold. However, the number of threshold values in a parameter set can vary. For example, in some embodiments, the upper and lower bounds of an ROC range may be specified instead of a single ROC threshold 406. The total number of parameter sets can also vary. For instance, instead of defining a single parameter set for all up-trending situations, the first parameter set 410 could be divided into multiple parameter sets, each with its own adjustment factor and corresponding threshold values. Likewise, the second parameter set 412 could be divided into multiple parameter sets such that the adjustment factor varies between different down-trending situations.

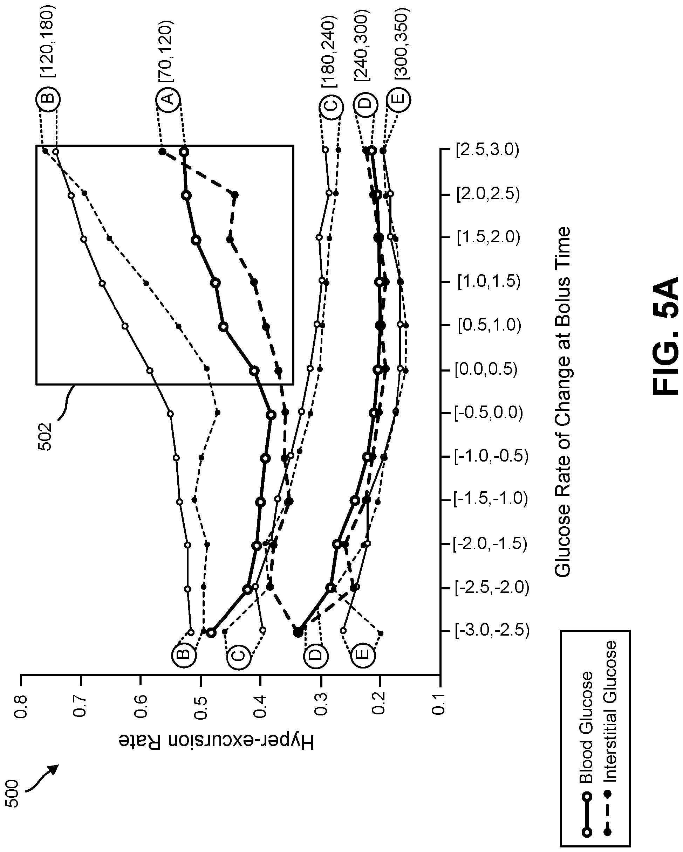

FIG. 5A is an example graph 500 of glucose hyper-excursion rate versus glucose ROC at bolus time. Hyper-excursion is when glucose rises to a level associated with hyperglycemia (e.g., above 180 mg/dL). The graph 500 contains five pairs of plots, labeled A to E. Each plot pair covers a different glucose range, includes a first plot of ROC calculated using SG values from an interstitial glucose sensor, and further includes a second plot of ROC calculated using BG values. As indicated by the interval notation in the figure (square brackets being inclusive, parentheses being exclusive), pair A covers glucose levels from [70-120), pair B covers [120-180), pair C covers [180-240), pair D covers [240-300), and pair E covers [300-350).

FIG. 5A shows that a bolus dose taken when glucose is rapidly increasing often leads to hyperglycemia. For example, a region 502 corresponds to rapid onset of hyperglycemia when a bolus dose is given at times of highly positive ROC even though glucose was below 180 mg/dL at the time of the dose. The doses given in conjunction with the measurements reflected in the region 502 were insufficient to counteract rising glucose level. In such situations, the dose could be increased to prevent or reduce the occurrence of hyperglycemia.

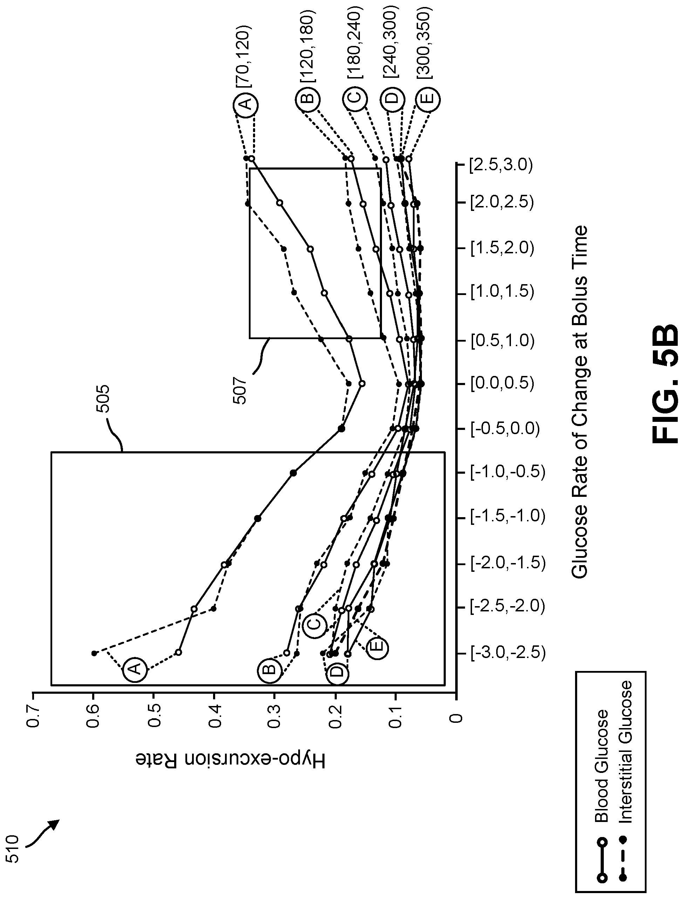

FIG. 5B is an example graph 510 of glucose hypo-excursion rate versus glucose ROC at bolus time. Hypo-excursion is when glucose falls to a level associated with hypoglycemia (e.g., below 70 mg/dL). The graph 510 contains five pairs of plots, which are labeled similarly as in the graph 500. FIG. 5B shows that a bolus dose given at times when glucose is changing rapidly often leads to hypoglycemia, regardless of the direction of change (rising or falling). For example, a region 505 corresponds to rapid onset of hypoglycemia when a bolus dose is given at times of highly negative ROC, and a region 507 corresponds to rapid onset of hypoglycemia when a bolus dose is given at times of highly positive ROC. The doses given in conjunction with the measurements reflected in the regions 505 and 507 were excessive in relation to the patient's actual insulin needs even though glucose was 70 mg/dL or greater at the time of the dose. In such situations, the dose could be decreased to prevent or reduce the occurrence of hypoglycemia.

One potential solution to address the problems illustrated in FIGS. 5A and 5B is to employ a universal, one-size-fits-all approach for everyone. Under a universal approach, insulin delivery can be adjusted by a predetermined amount (e.g., a fixed percentage) when glucose levels are changing by more than a threshold amount. For example, the dose could be increased by 10% when the magnitude of the ROC exceeds a first threshold in the positive direction and decreased by 10% when the magnitude of the ROC exceeds the first threshold in the negative direction. However, this universal approach can still result in inaccurate dosing by failing to account for differences in lifestyles and physiologies among people.

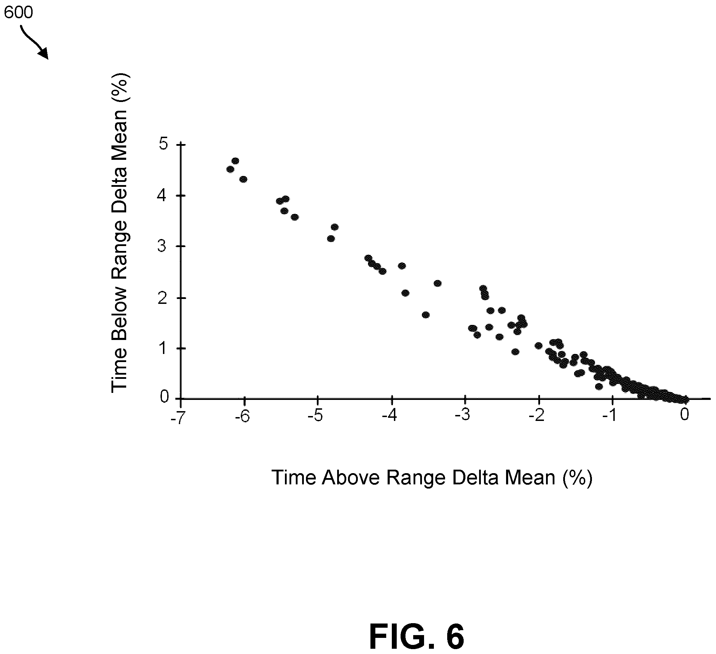

FIG. 6 is a graph 600 of the average change in time-above-range (TAR) versus the average change in time-below-range (TBR) for a sample patient population when the same set of adjustment parameters is applied across the entire patient population. The graph 600 shows that a tradeoff exists between TAR and TBR across the population. A dose that decreases TBR tends to increase TAR. Likewise, a dose that decreases TAR tends to increase TBR. There is no dose that simultaneously minimizes TAR and TBR for all people. Consequently, universal approaches are usually conservative with respect to the extent of the adjustments and the conditions under which adjustments are made. For instance, adjustments may be applied infrequently (e.g., ten times over a year or longer) and may involve changing the dosage amount by less than would be optimal for the recipient.

Instead of a universal approach, a person-specific approach may be applied to adjust dosage (e.g., bolus doses) when glucose levels are rapidly changing. As mentioned above, the physiological model(s) 138 can include one or more models specific to the dose recipient, e.g., person 105, and can be used to determine a set of adjustment parameters through simulating glucose level under different scenarios. The scenarios simulated can include different starting glucose values (e.g., SG at dose time), different rates of change, different dosage amounts, and/or other factors that affect the recipient's glucose level.

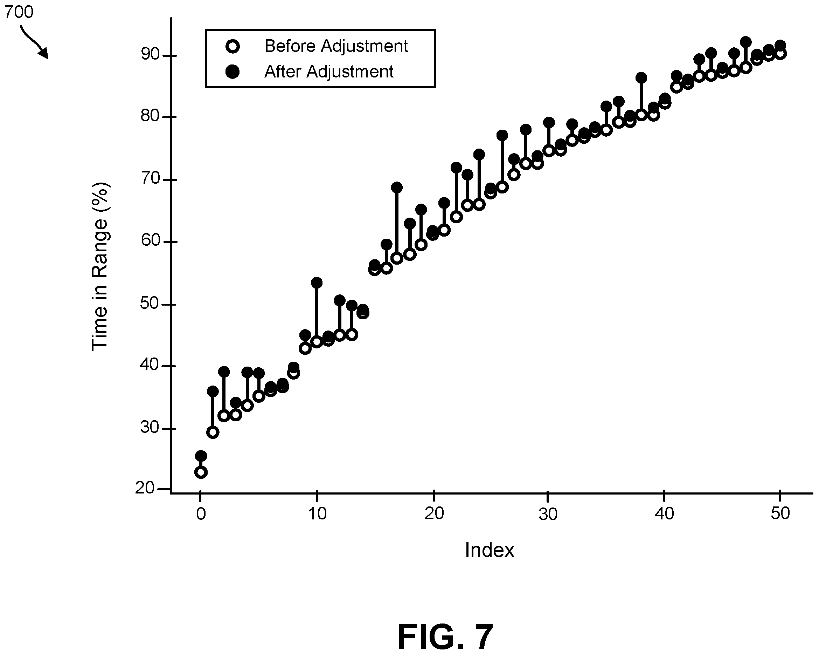

FIG. 7 is a graph 700 of time-in-range (TIR) versus sample index for fifty measurement samples from a representative diabetes patient. Each measurement sample corresponds to a separate dose. The graph 700 shows an increase in TIR as a result of applying person-specific adjustments using adjustment parameters derived in accordance with the techniques described herein. For purposes of the present discussion, TIR may refer to the duration in which glucose levels are euglycemic (e.g., 70-180 mg/dL, in particular, 100-120 mg/dL). However, TIR can be time spent in any target range. The boundaries of the target range depend on the physiological condition at issue. In the example of FIG. 7, applying person-specific adjustments resulted in an average TIR increase of approximately 3% compared to delivering an unadjusted dose.

The results in the graph 700 demonstrate that TIR is a suitable metric for evaluating potential adjustment parameter values. Accordingly, in some implementations, adjustment parameters are determined using TIR as a selection criterion. For example, candidate parameter sets may be formed, where each candidate parameter set includes a different combination of adjustment factor, measurement threshold, and ROC threshold values. Each candidate parameter set can then be evaluated through simulation to determine whether the candidate parameter set leads to an increase in TIR without also increasing time-below-range (TBR). Any candidate parameter set that results in increased TBR can be excluded from further consideration. However, some implementations may allow TBR to increase by a certain amount so long as TIR increases. For instance, prior to running the simulation to determine the parameter sets 410 and 412 for the person 105, the computing system 130 may request user input confirming whether increased TBR is acceptable to the person 105. Other selection criteria or optimization strategies can be used to determine which adjustment parameter values to use for any particular person.

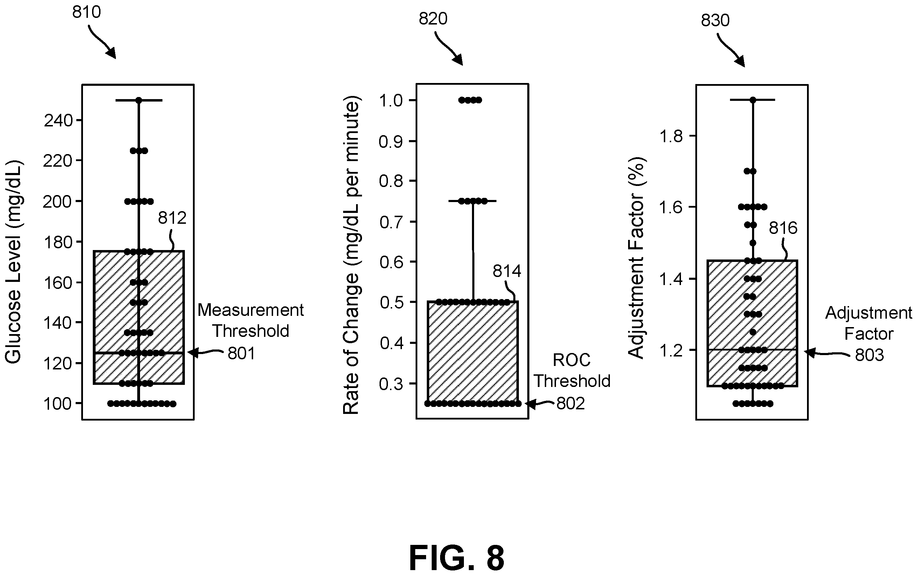

FIG. 8 includes three graphs (810, 820, and 830) showing adjustment parameter values that were evaluated to determine an adjustment parameter set for the adjustments in FIG. 7. For the sake of simplicity, the example of FIG. 8 is limited to uptrend parameters, e.g., the parameter set 410 in FIG. 4. However, similar techniques may be used to determine downtrend parameters. In this example, the adjustment parameters were initially grouped into 74 candidate parameter sets and simulated to determine that 51 of the 74 candidate parameter sets (the shaded regions) resulted in increased TIR without increasing TBR. The remaining 23 parameter sets either failed to increase TIR or increased TIR while also increasing TBR, and where thus excluded from consideration. The adjustment parameters ultimately selected include a measurement threshold 801, an ROC threshold 802, and an adjustment factor 803.

The simulation was performed using a glucose model derived from measurements performed on the representative diabetes patient. For example, SG and/or BG values were recorded together with corresponding insulin doses given at the time of the glucose measurements. Parameters of the glucose model (e.g., the value of SI and other parameters discussed above with respect to the model in FIG. 2) were then determined consistent with the recorded data. The glucose model was then used to simulate glucose level under different combinations of adjustment parameter values (measurement threshold, ROC threshold, and adjustment factor). Each combination represents a candidate parameter set that was simulated to determine a corresponding TIR and TBR. The results were then compared to determine which candidate parameter sets met the criteria of increased TIR without increased TBR.

Graph 810 shows the distribution of glucose level values that formed candidates for the measurement threshold 801. The glucose levels range from 100-250 mg/dL. The measurement threshold 801 has a value of 125 mg/dL and was determined as being the median value within a group 812 of glucose levels associated with candidate parameter sets that satisfied the above-mentioned criteria.

Graph 820 shows the distribution of ROC values that formed candidates for the ROC threshold 802. The ROC values range from 0.2-1.0 mg/dL per minute. The ROC threshold 802 has a value of 0.25 mg/dL/min and was determined as being the median value within a group 814 of ROC values associated with candidate parameter sets that satisfied the above-mentioned criteria.

Graph 830 shows the distribution of adjustment factor values that formed candidates for the adjustment factor 803. The adjustment factor values correspond to scaling factors that provide for an increase between 10-90% (1.1 to 1.9). However, adjustment factors are not limited to scaling factors and can be any numerical value that modifies a dosage amount. For instance, an adjustment factor could be an offset value that is summed with an initial dosage. The adjustment factor 803 has a value of 1.2 (corresponding to a 20% increase) and was determined as being the median value within a group 816 of adjustment factor values associated with candidate parameter sets that satisfied the above-mentioned criteria.

Although the measurement threshold 801, the ROC threshold 802, and the adjustment factor 803 were calculated as median values, other methods of determining the adjustment parameter values are possible. For instance, the adjustment parameters may be calculated as average values in some implementations. As another example, instead of considering each adjustment parameter separately, candidate parameter sets may be plotted together within the overall search space (e.g., as three-dimensional points) to compute a centroid with coordinates corresponding to the measurement threshold 801, the ROC threshold 802, and the adjustment factor 803.

FIG. 8 is merely an example to show how an adjustment parameter set can be determined based on the results of a simulation. The values discussed in connection with this example are illustrative and non-limiting. As such, the search space may be different depending on implementation and/or the physiological condition at issue. For example, in some implementations, the search space for adjustment parameters applied to insulin doses may encompass the following value ranges:

Measurement (glucose) threshold: 100-350 mg/dL (uptrend) or 40-150 mg/dL (downtrend),

ROC threshold: 0.25 to 2.5 mg/dL/min (uptrend) or -0.25 to -2.5 mg/dL/min (downtrend), and

Adjustment factor: 5-90% increase (uptrend) or 5-90% decrease (downtrend).

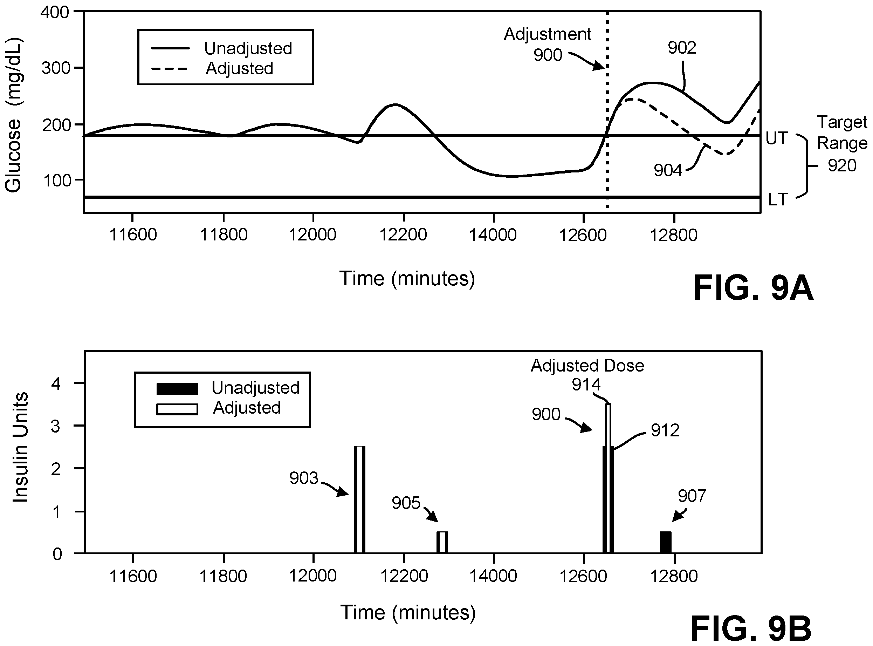

**FIG. 9A and 9B** show example results of adjusting an insulin dose. FIG. 9A is a comparison between a plot 902 of glucose in the absence of adjustments and a plot 904 of glucose when an adjustment 900 is applied. FIG. 9B is a bar graph showing the difference between the amounts of an unadjusted dose 912 and an adjusted dose 914 corresponding to the adjustment 900. As indicated in FIG. 9A, the glucose response is essentially identical leading up to the adjustment 900 since earlier doses 903 and 905 were left unadjusted. The adjustment 900 was applied based on a measurement threshold of 100 mg/dL, an ROC threshold of 0.25, and an adjustment factor of 1.55 (a 55% increase).

The adjustment 900 increased the dose 912 by approximately one unit and was applied at a time when glucose was rising rapidly, i.e., ROC above 0.25. The adjusted dose 914 caused a return to a target range 920. In this example, the target range 920 was set with a lower threshold (LT) and an upper threshold (UT) of 70 and 180 mg/dL, respectively. The return to the target range 920 occurred after a subsequent dose 907, which was left unadjusted since the threshold conditions for the adjustment 900 were not present at the time of the subsequent dose 907. In contrast, the plot 902 shows that glucose levels failed to return to the target range 920 even after the subsequent dose 907. TIR across the entire measurement period was 57.3 in the case of no adjustment and 68.6 with the adjustment 900. Accordingly, this example demonstrates that adjustments can be applied to facilitate a return to target range and/or increased time-in-range.

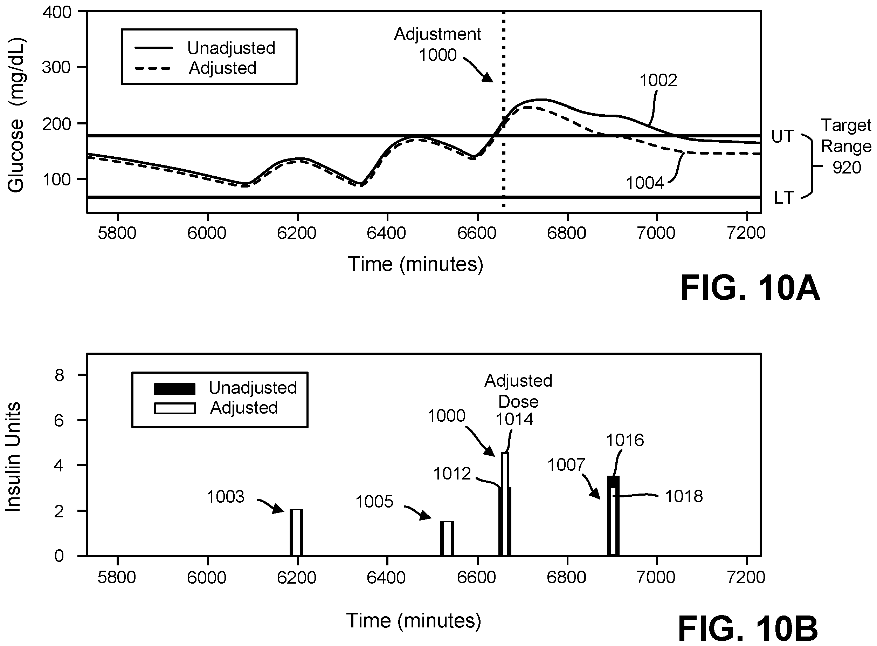

**FIG. 10A and 10B** show another example of the results of adjusting an insulin dose. FIG. 10A is a comparison between a plot 1002 of glucose in the absence of adjustments and a plot 1004 of glucose when an adjustment 1000 is applied. FIG. 10B is a bar graph showing the difference between the amounts of an unadjusted dose 1012 and an adjusted dose 1014 corresponding to the adjustment 1000. As indicated in FIG. 10A, the glucose response is essentially identical leading up to the adjustment 1000 since earlier doses 1003 and 1005 were left unadjusted. The adjustment 1000 was applied based on a measurement threshold of 160 mg/dL, an ROC threshold of 0.75, and an adjustment factor of 1.60 (a 60% increase). The adjustment 1000 increased the dose 1012 by approximately 2 units and was applied at a time when glucose was rising rapidly, i.e., ROC above 0.75. In contrast to the example in FIGS. 9A and 9B, here the glucose levels did eventually return to the target range 920 even without adjustments. However, as indicated by the plot 1004, the adjustment 1000 led to a faster return to the target range 920.

Additionally, the adjustment 1000 enabled a subsequent dose 1007 to be delivered using less insulin. Specifically, the subsequent dose 1007 involved an insulin amount 1018 lower than a corresponding original amount 1016. However, the insulin amount 1018 was not computed by applying an adjustment factor. Instead, both amounts 1016 and 1018 were determined using the same dosing formula and based on prevailing conditions at the time of the subsequent dose 1007. The difference between the original amount 1016 and the insulin amount 1018 is due to the fact that in the adjustment case (plot 1004), glucose levels had already fallen to the target range 920 by the time of the subsequent dose 1007, whereas glucose was still out of range in the unadjusted case (plot 1002). Accordingly, adjusting a dose can influence the amount of a subsequent dose even if an adjustment factor is not applied to the subsequent dose.

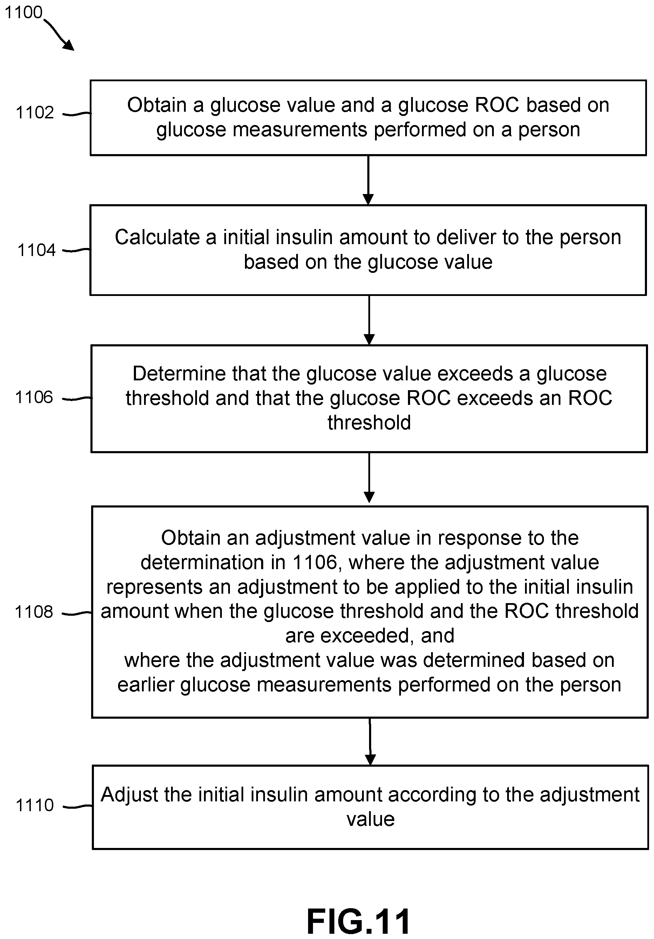

**FIG. 11** is a flow diagram of a process 1100 for adjusting dosage according to some embodiments. The processes depicted in FIG. 11 and subsequent flow diagrams are described with respect to insulin dosage and glucose level but can also be applied to dosing in other therapy contexts. The process 1100 can be performed on a computing device having access to one or more sets of adjustment parameters. For instance, the process 1100 may involve operations performed by a processing unit(s) of a monitoring device such as the monitoring device 110 in FIG. 1.

At 1102, a glucose value and a glucose ROC are obtained based on glucose measurements performed on a person (e.g., the person 105). The glucose value may correspond to a most recent measurement of the person's glucose level. The glucose ROC can be determined from two or more recent measurements. For instance, the glucose ROC may be calculated by subtracting the value of the immediately preceding measurement from the value of the most recent measurement and dividing the difference by the time interval between these measurements. Other methods of determining ROC are possible including, for example, as an average rate of change over three or more successive measurements. The glucose measurements are typically performed using an interstitial glucose sensor but can include measurements from other types of sensors, e.g., a blood glucose meter.

At 1104, an initial insulin amount is calculated for delivery to the person, based on the glucose value obtained in 1102. The initial insulin amount can be calculated based on a dosing formula such as Equation 1. The dosing formula may utilize the glucose value from 1102 in combination with ISF, ICR, and other factors to compute the initial insulin amount but generally does not take ROC into consideration.

At 1106, it is determined that the glucose value exceeds a glucose threshold, and that the glucose ROC exceeds an ROC threshold. The glucose threshold and the ROC threshold can be specified as part of the same adjustment parameter set and may, for example, correspond to the measurement threshold 404 and the ROC threshold 406 in parameter set 410 or the parameter set 412 of FIG. 4.

At 1108, an adjustment value is obtained in response to the determination in 1106. The adjustment value represents an adjustment to be applied to the initial insulin amount when the glucose threshold and the ROC threshold are exceeded. For instance, the adjustment value may correspond to the adjustment factor 402 from the same parameter set as the glucose threshold and the ROC threshold. The adjustment value provides for an increase or decrease in the initial insulin amount and can be expressed as a scaling factor. In contrast to the initial insulin amount, the adjustment value is predetermined based on earlier glucose measurements performed on the same person, i.e., prior to the measurements in 1102. The earlier glucose measurements can be performed using the same or different sensors. For example, the earlier glucose measurements may have been recorded over one or two weeks, using an earlier instance of the monitoring device that obtained the measurements in 1102. The adjustment value can be retrieved from a memory associated with the computing device performing the process 1100, e.g., the memory subsystem 118 of the monitoring device 110. The adjustment value may have been stored in association with the glucose threshold and ROC threshold from 1106, e.g., as part of the same adjustment parameter set.

At 1110, the initial insulin amount is adjusted according to the adjustment value. The insulin dose can be delivered automatically by an insulin delivery device (e.g., the delivery device 120) or manually by a user of an insulin delivery device (e.g., the person 105). The adjustment may involve communicating an adjusted insulin amount to the insulin delivery device or user, thereby causing delivery in the adjusted insulin amount instead of the initial insulin amount. In some instances, the initial insulin amount may also be communicated.

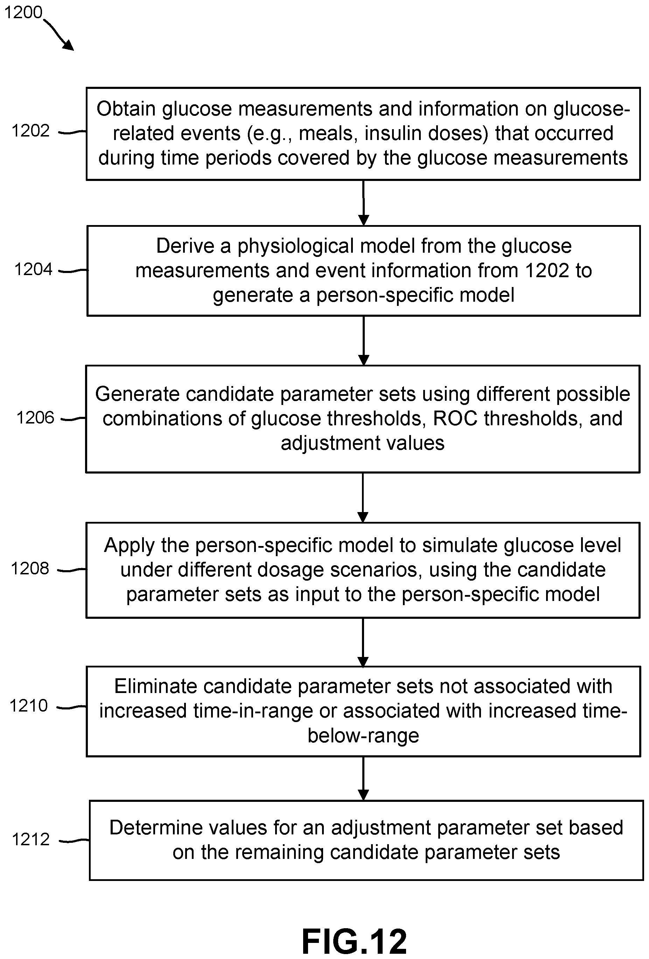

**FIG. 12** is a flow diagram of a process 1200 for determining adjustment parameters according to some embodiments. The process 1200 can be performed on a computing device or system having access to historical glucose measurements. For instance, the process 1200 may involve operations performed by a processing unit(s) of a computing system such as the computing system 130 in FIG. 1.

At 1202, glucose measurements and information on glucose-related events are obtained. The glucose measurements can be performed by one or more sensors (e.g., an interstitial glucose sensor and/or a blood glucose meter) and may be collected over the course of several days, weeks, or months. As such, the glucose measurements may include SG and/or BG values obtained from the one or more sensors. For example, the computing system 130 may receive the glucose measurements and information on glucose-related events from one or more monitoring devices that collect measurements from sensors used on the person. Glucose-related events can include insulin doses (e.g., delivery times and dose amount), meals (e.g., mealtimes and number of carbohydrates in a meal), and other events that influence glucose level.

At 1204, a physiological model is derived from the glucose measurements and event information obtained in 1202 as a person-specific model usable for estimating glucose level. In particular, the person-specific model may be configured to estimate the person's glucose level at a particular time in the future based on information about the person's current state (e.g., current glucose level and glucose ROC) and further based on information about glucose-related events that have occurred or are expected to occur (e.g., an upcoming basal dose). The derivation can involve fitting a generic physiological model and may result in one or more model parameters being customized according to the person's unique physiology and behavior, such that the glucose estimates output by the person-specific model are consistent with the glucose measurements and event information from 1202.

At 1206, candidate parameter sets are generated using different possible combinations of glucose thresholds, ROC thresholds, and adjustment values. For example, each candidate parameter set may include values for the adjustment factor 402, the measurement threshold 404, and the ROC threshold 406, and each candidate parameter set may differ from every other candidate parameter set with respect to the value of at least one of these three parameters. Therefore, each candidate parameter set can include a unique combination of parameter values representing the conditions under which an adjustment could potentially be applied. The candidate parameter sets can be randomly generated. Referring back to the example discussed in conjunction with FIG. 8, one or more candidate parameter sets may be formed to include a random measurement threshold between 100-350 mg/dL, a random ROC threshold between 0.25 to 2.5, and/or a random adjustment factor between 5-90%. Alternatively or additionally, the candidate parameter sets may be generated as variations of observed conditions. For instance, the measurements and event information from 1202 may indicate the glucose value and glucose ROC at the time of an actual dose along with the dose amount. One or more candidate parameter sets could then be generated by changing the glucose value, the glucose ROC, and/or the dose amount to form a hypothetical scenario. Multiple candidate parameter sets can be generated from observations regarding a single dose.

At 1208, the person-specific model is applied to simulate the glucose level of the person under different dosage scenarios. In particular, the candidate parameter sets from 1206 are input to the person-specific model to estimate the person's glucose response to insulin doses delivered using different dosage amounts and under different glucose levels and different rates of change. The simulated dosage amounts can be computed based on the adjustment values in the candidate parameter sets, e.g., a bolus dose computed according to Equation 1 and modified in accordance with the value of an adjustment factor 402. The estimated glucose levels can cover a certain duration after the delivery of each simulated dose, e.g., the next 30 minutes or up to the next expected dose. In this manner, the dosage scenarios can be evaluated based on TIR, TAR, TBR, and/or other metrics relating to change in glucose over time. Further, in some instances, the duration may span multiple doses including the simulated dose and one or more subsequent doses.

At 1210, candidate parameter sets that are not associated with increased TIR or that are associated with increased TBR are eliminated, leaving a subset of candidate parameter sets remaining. Therefore, only dosage scenarios that are correlated with improved outcomes relative to delivery of an unadjusted dose under the same circumstances may be considered.

At 1212, the values for an adjustment parameter set (e.g., parameter set 410 or parameter set 412) are determined based on the remaining candidate parameter sets. The values for the adjustment parameter set can be determined in various ways, for example, through computing each adjustment parameter as a median or average value, as discussed above in connection with FIG. 8. The adjustment parameter set determined in 1212 provides for individualized dosing and can be applied to determine a more optimal dose under a variety of dosage scenarios. Based on the results of the simulation in 1208, the adjusted insulin amounts are expected to maximize time-in-range relative to other (e.g., unadjusted) insulin amounts whenever the glucose threshold and the ROC threshold are exceeded at the same time. When the glucose threshold or the ROC threshold are not exceeded, the dose can be delivered without adjustment. Therefore, the insulin needs of the person can be met using a combination of adjusted doses and unadjusted doses.

**FIG. 13** is a flow diagram of a process 1300 for overriding an adjustment according to some embodiments. The process 1300 can be performed on a computing device or system having access to one or more adjustment parameter sets. For instance, the process 1300 may involve operations performed by a processing unit(s) of a monitoring device, e.g., the monitoring device 110 after receiving the adjustment parameters 304 from the computing system 130. The process 1300 involves a safety check on an adjustment and can be performed to deliver an initial insulin amount instead of an adjusted insulin amount when it is determined that the adjusted insulin amount would lead to a worse outcome than the initial insulin amount.

At 1302, a glucose value and a glucose ROC are obtained based on glucose measurements performed on a person (e.g., the person 105). The functionality in 1302 can be implemented in a similar manner as in 1102 of FIG. 11.

At 1304, an initial insulin amount is calculated for delivery to the person, based on the glucose value obtained in 1302. The calculation in 1304 can be performed in a similar manner as the calculation in 1104 of FIG. 11, e.g., using a dosing formula.

At 1306, an adjusted insulin amount is determined. The adjusted insulin amount represents an increase or decrease in the initial insulin amount calculated in 1304 and can be determined using an adjustment parameter set applicable to the glucose value and glucose ROC obtained in 1302. For example, the adjusted insulin amount may be determined through applying an adjustment value based on the glucose value exceeding a glucose threshold and further based on the glucose ROC exceeding an ROC threshold, as discussed above in reference to FIG. 11, 1106 and 1108. However, the process 1300 can be used to override any planned adjustment to a dose irrespective of how the adjusted amount is determined.

At 1308, future glucose levels are simulated using a person-specific physiological model. The person-specific model may have been generated using earlier glucose measurements performed on the same person. The model used for the simulation in 1308 can be the same model that was used to determine the adjustment parameters for the adjusted insulin amount in 1306. For example, referring back to FIG. 12, the computing system 130 may have communicated model parameters to the monitoring device 110 (or another computing device in the system) after deriving the model in 1204. As such, the monitoring device 110 or other computing device may be able to perform a similar simulation as the simulation in 1208 of FIG. 12.

The simulation in 1308 may have a more limited scope compared to a simulation used to determine adjustment parameters. For instance, instead of simulating glucose levels under a variety of possible dosage scenarios, the simulation in 1308 may be limited to predicting glucose changes that would occur after delivery of the adjusted insulin amount in 1306. Additionally, the simulation in 1308 may involve a shorter time frame (e.g., glucose levels within the next 30 minutes), whereas the simulation used to determine adjustment parameters could involve a longer time frame (e.g., the next several hours or up to the next dose). Thus, the simulation in 1308 would typically be focused on the present state of the person and the potential effects of the adjustment. The glucose levels simulated in 1308 would therefore include glucose levels representing a predicted result of delivering the adjusted insulin amount in conjunction with the glucose value and glucose ROC obtained in 1302.

The simulation in 1308 may also be different in scope because it uses more recent information (e.g., the glucose measurements in 1302), which was not necessarily available at the time of the simulation used to determine adjustment parameters. Moreover, even if the earlier simulation covered the same conditions (e.g., the same glucose value and glucose ROC as in 1302), the adjustment parameters would have been determined across multiple dosage scenarios rather than being optimized for a single scenario. As discussed above in reference to FIG. 8, the measurement threshold 801, ROC threshold 802, and adjustment factor 803 are determined based on multiple candidate parameter sets associated with improved TIR. Consequently, although the adjusted insulin amount in 1306 is generally expected to lead to longer TIR compared to the initial insulin amount calculated in 1304, this is not always the case. For various reasons, there may be times when the initial insulin amount would perform better than the adjusted insulin amount. Changes in the physiology or behavior of the person (e.g., weight gain, switch to a more sedentary lifestyle, missed meals) or incorrect user input (e.g., underestimating the number of carbohydrates in a meal) can contribute to skewed dosing such that the initial insulin amount would actually be more appropriate for the next dose. Therefore, it may be beneficial to confirm that the adjusted insulin amount would work better before delivering a dose in the adjusted amount.

At 1310, it is determined that one or more of the future glucose levels from the simulation in 1308 exceed a threshold associated with a target glucose range. The future glucose levels can be evaluated with respect to the upper threshold (e.g., 180 mg/dL) and/or the lower threshold (e.g., 70 mg/dL) of the target glucose range. For example, if the glucose value in 1302 is already close to the upper threshold, excursions above the upper threshold could be ignored. Similarly, if the glucose value in 1302 is close to the lower threshold, excursions below the lower threshold could be ignored. Thus, hyper-excursions and/or hypo-excursions may be used as a heuristic for concluding that the adjusted insulin amount would perform worse than the initial insulin amount.

The strictness with which the determination in 1310 is made can vary. In some implementations, any excursion (i.e., a single future glucose value) beyond the target glucose range may be sufficient for the adjusted insulin amount to be deemed invalid. Alternatively, the criteria could be relaxed by requiring that a threshold number of future glucose values go beyond the target glucose range, e.g., ten excursions in the same direction. As another example, the distribution of the excursions could be considered, e.g., so as to require ten excursions in succession or across a certain sized block of time/sample count.

Optionally, the simulation in 1308 may also involve predicting a result of delivering the initial insulin amount, thus allowing for direct comparison between the initial and adjusted amounts (e.g., based on TIR). This involves more computation but adds to the set of criteria that can be applied to rule out use of the adjusted insulin amount.

At 1312, in response to the determination in 1310, the initial insulin amount is caused to be delivered instead of the adjusted insulin amount. Delivery of the initial insulin amount can be realized in a number of ways. For example, the computing device making the determination in 1310 may hold off on communicating the dosage to a delivery device until the determination is complete. Alternatively, the computing device could communicate the initial insulin amount as a follow-up to a communication of the adjusted insulin amount. In some implementations, the delivery device may receive an override command or signal indicating that the adjusted insulin amount is not to be used.

The simulation in 1308 and the determination in 1310 can be repeated for subsequent planned adjustments, e.g., anytime an initial insulin amount is adjusted in accordance with the operations shown in FIG. 11. Adjustments can be overridden on a case-by-case basis depending on the result of the determination in 1310. The overriding of an adjustment can be performed without updating or changing the existing adjustment parameters. As discussed above, the simulation in 1308 is typically focused on the present state of the person and the potential effects of the adjustment rather than possible dosage scenarios. Updating of the adjustment parameters may not be appropriate in such situations.

Although the adjustment parameters may not be updated in conjunction with overriding of adjustments, the adjustment parameters can be updated occasionally to take into consideration newer measurements. For instance, the computing system 130 may perform the process 1200 of FIG. 12 periodically, e.g., once a month, using glucose measurements and event information communicated to the computing system 130 from the monitoring device 110. In turn, each time the computing system 130 determines new values for an adjustment parameter set, the computing system 130 can communicate those values to the monitoring device 110 for use with subsequent dosing.

Adjustment parameters can also be updated on-demand in response to a user request. For instance, when the monitoring device 110 receives user input of updated information about the person 105 (e.g., a new ISF or ICR value), the monitoring device 110 can prompt the user for additional input indicating whether the adjustment parameters should be updated. If the user indicates that they want to update the adjustment parameters, the monitoring device 110 can request the computing system 130 to rerun the simulation in 1208. In some instances, the adjustment parameters may be updated in conjunction with updating of a physiological model, e.g., when the physiological model is refitted in accordance with the functionality in FIG. 12, 1204.

**FIG. 14** depicts an example therapy delivery system 1400 for a person 1401. Similar to the system 100 in FIG. 1, the system 1400 may be an insulin delivery system. Accordingly, functionality or features described with respect to components of the system 100 may also be provided through analogous components in the system 1400. Likewise, components of the system 100 may incorporate functionality or features described with respect to their counterparts in the system 1400. However, it is intended that any aspect, embodiment, or description relating to an insulin delivery system shall be applicable to a therapy delivery system which delivers a therapy other than insulin.

As shown in FIG. 14, the therapy delivery system 1400 includes a delivery device 1402, a monitoring device 1404, a computing device 1406, and an optional remote or cloud computing system 1408. Delivery device 1402 may correspond to the delivery device 120 in FIG. 1. Monitoring device 1404 may correspond to the monitoring device 110. Computing device 1406 may correspond to the computing system 130 or a device in communication with the computing system 130. For example, the remote/cloud computing system 1408 may take the role of the computing system 130, and the computing device 1406 may operate as a trusted intermediary facilitating secured (e.g., encrypted) communications between the computing system 1408 and other components such as the delivery device 1402 and/or the monitoring device 1404.

The delivery device 1402, the monitoring device 1404, and the computing device 1406 may be embodied in various ways, including being disposed in one or more device housings. For example, in some embodiments, all of the devices 1402-1406 may be disposed in a single device housing. In some embodiments, each of the devices 1402-1406 may be disposed in a separate device housing. In some embodiments, two or more of the devices 1402-1406 may be disposed in the same device housing, and/or a single device 1402, 1404, or 1406 may have two or more parts that are disposed in two or more housings. Such embodiments, and combinations thereof, are contemplated to be within the scope of the present disclosure.

FIG. 14 also depicts communications links 1412-1418. The communications links 1412-1418 may each be a wired connection and/or a wireless connection. In the case where two devices are located in the same device housing, the communication link may include, for example, wires, cables, and/or communication buses on a printed circuit board, among other things. In the case where two devices are separated from each other in different device housings, the communication links may be wired and/or wireless connections. Wired connections may include, without limitation, an Ethernet connection, a Universal Serial Bus (USB) connection, and/or another type of physical connection. Wireless connections may include, without limitation, a cellular connection, a Wi-Fi connection, a Bluetooth® connection, a mesh network connection, and/or another type of connection using a wireless communication protocol. Some embodiments of the communication links 1412-1418 may use direct connections, such as Bluetooth® connections, and/or may use connections that route through one or more networks or network devices (not shown), such as an Ethernet network, a Wi-Fi network, a cellular network, a satellite network, an intranet, an extranet, the Internet, and/or the Internet backbone, among other types of networks. Various combinations of wired and/or wireless connections may be used for the communication links 1412-1418.

Aspects of the insulin delivery system 1400 are described below. Further aspects and details may be described in United States Patent Nos.: 4,562,751; 4,685,903; 5,080,653; 5,505,709; 5,097,122; 6,485,465; 6,554,798; 6,558,320; 6,558,351; 6,641,533; 6,659,980; 6,752,787; 6,817,990; 6,932,584; and 7,621,893. The entire contents of each of the foregoing United States Patents are hereby incorporated by reference herein.

The delivery device 1402 is configured to deliver a therapeutic substance (e.g., insulin) to a person 1401. The delivery device 1402 may be secured to the person 1401 (e.g., to the body or clothing of the person 1401) or may be implanted on or in the body of the person 1401. In some embodiments, the delivery device 1402 may include a reservoir, an actuator, a delivery mechanism, and a cannula (not shown). The reservoir may be configured to store an amount of the therapeutic substance. In some embodiments, the reservoir may be refillable or replaceable. The actuator may be configured to drive the delivery mechanism. In some examples, the actuator may include a motor, such as an electric motor. The delivery mechanism may be configured to move the therapeutic substance from the reservoir through the cannula. In some examples, the delivery mechanism may include a pump and/or a plunger. The cannula may facilitate a fluidic connection between the reservoir and the body of the person 1401. The cannula and/or a needle may facilitate delivery of the therapeutic substance to a tissue layer, vein, or body cavity of the person 1401. During operation, the actuator, in response to a signal (e.g., a command signal), may drive the delivery mechanism, thereby causing the therapeutic substance to move from the reservoir, through the cannula, and into the body of the person 1401.

The components of the delivery device 1402 described above are merely provided as examples. The delivery device 1402 may include other components, such as, without limitation, a power supply, a communication transceiver, computing resources, and/or user interfaces, among other things. Persons skilled in the art will recognize various implementations of the delivery device 1402 and the components of such implementations. All such implementations and components are contemplated to be within the scope of the present disclosure.

Monitoring device 1404 is configured to detect a physiological condition (e.g., a glucose concentration level) of the person 1401 and may also be configured to detect other things. The monitoring device 1404 may be secured to the body of the person 1401 (e.g., to the skin of person 1401 via an adhesive) and/or may be at least partially implanted into the body of the person 1401. Depending on the particular location or configuration, the monitoring device 1404 may be in contact with biological matter (e.g., interstitial fluid and/or blood) of the person 101.

The monitoring device 1404 includes one or more sensors (not shown), such as, without limitation, electrochemical sensors, electrical sensors, and/or optical sensors. As persons skilled in the art will understand, an electrochemical sensor may be configured to respond to the interaction or binding of a biological marker to a substrate by generating an electrical signal based on a potential, conductance, and/or impedance of the substrate. The substrate may include a material selected to interact with a particular biomarker, such as glucose. The potential, conductance, and/or impedance may be proportional to a concentration of the particular biomarker. In the case of electrical sensors, and as persons skilled in the art will understand, an electrical sensor may be configured to respond to an electrical biosignal by generating an electrical signal based on an amplitude, frequency, and/or phase of the electrical biosignal. The electrical biosignal may include a change in electric current produced by the sum of an electrical potential difference across a tissue, such as the nervous system, of the person 1401. In some embodiments, the electrical biosignal may include portions of a potential change produced by the heart of the person 1401 over time, e.g., recorded as an electrocardiogram, that are indicative of a glucose level of the person 1401. In the case of optical sensors, as persons skilled in the art will understand, an optical sensor may be configured to respond to the interaction or binding of a biological marker to a substrate by generating an electrical signal based on change in luminance of the substrate. For example, the substrate may include a material selected to fluoresce in response to contact with a selected biomarker, such as glucose. The fluorescence may be proportional to a concentration of the selected biomarker.

In some embodiments, the monitoring device 1404 may include other types of sensors that may be worn, carried, or coupled to the person 1401 to measure activity of the person 1401 that may influence the glucose levels or glycemic response of the person 1401. As an example, the sensors may include an acceleration sensor configured to detect an acceleration of the person 1401 or a portion of the person 1401, such as the person's hands or feet. The acceleration (or lack thereof) may be indicative of exercise, sleep, or food/beverage consumption activity of the person 101, which may influence the glycemic response of the person 1401. In some embodiments, the sensors may include heart rate and/or body temperature, which may indicate an amount of physical exertion experienced by the person 1401. In some embodiments, the sensors may include a Global Positioning System (GPS) receiver which detects GPS signals to determine a location of the person 1401.

The sensors described above are merely provided as examples. Other sensors or types of sensors for monitoring physiological condition, activity, and/or location, among other things, will be recognized by persons skilled in the art and are contemplated to be within the scope of the present disclosure. For any sensor, the signal provided by a sensor shall be referred to as a "sensor signal."

The monitoring device 1404 may include components and/or circuitry configured to pre-process sensor signals. Pre-processing may include, without limitation, amplification, filtering, attenuation, scaling, isolation, normalization, transformation, sampling, and/or analog-to-digital conversion, among other things. Persons skilled in the art will recognize various implementations for such pre-processing, including, without limitation, implementations using processors, controllers, integrated circuits, application specific integrated circuits (ASICs), hardware, firmware, programmable logic devices, and/or machine-executable instructions, among others. The types of pre-processing and their implementations are merely provided as examples. Other types of pre-processing and implementations are contemplated to be within the scope of the present disclosure. In some embodiments, the monitoring device 1404 may not perform pre-processing.

As used herein, the term "sensed data" shall mean and include the information represented by a sensor signal or by a pre-processed sensor signal. In some embodiments, sensed data may include glucose levels in a person 1401, acceleration of a part of the person 1401, heart rate of the person 1401, temperature of the person 1401, and/or geolocation (e.g., GPS location) of the person 1401, among other things. The monitoring device 1404 may communicate sensed data to the delivery device 1402 via communication link 1412 and/or to the computing device 1406 via communication link 1414. Use of sensed data by the delivery device 1402 and/or by the computing device 1406 will be described later herein.

The computing device 1406 provides processing capabilities and may be implemented in various ways. In some embodiments, the computing device 1406 may be a consumer device, such as a smartphone, a computerized wearable device (e.g., a smartwatch), a tablet computer, a laptop computer, or a desktop computer, among others, or may be a special purpose device (e.g., a portable control device) provided by, for example, the manufacturer of the delivery device 1402. In some embodiments, the computing device 1406 may be "processing circuitry" (defined below) that is integrated with another device, such as the delivery device 1402. In some embodiments, the computing device 1406 may be secured to the person 1401 (e.g., to the body or clothing of person 1401), may be at least partially implanted into the body of person 101, and/or may be held by the person 1401.

For each of the embodiments of the computing device 1406, the computing device 1406 may include various types of logic circuitry, including, but not limited to, microprocessors, controllers, digital signal processors (DSPs), application specific integrated circuits (ASICs), field-programmable gate arrays (FPGAs), central processing units (CPU), graphics processing units (GPU), programmable logic devices, memory (e.g., random access memory, volatile memory, non-volatile memory, etc.), or other discrete or integrated logic circuitry, as well as combinations of such components. The term "processing circuitry" may generally refer to any of the foregoing logic circuitry, alone or in combination with other logic circuitry, or any other circuitry for performing computations.

Aspects of the delivery device 1402, the monitoring device 1404, and the computing device 1406 have been described above. One or more of the devices 1402-1406 may include a user interface (not shown) that presents information to the person 1401 and/or receives information from the person 1401. The user interface may include a graphical user interface (GUI), a display device, a keyboard, a touchscreen, a speaker, a microphone, a vibration motor, buttons, switches, and/or other types of user interfaces. Persons skilled in the art will recognize various types of user interfaces that may be used, and all such user interfaces are contemplated to be within the scope of the present disclosure. For example, where the computing device 1406 is a consumer device such as a smart phone, tablet computer, laptop computer, or the like, the user interfaces would include a display device, a physical and/or virtual keyboard, and/or audio speakers provided by such consumer devices, among other things. In some embodiments, a user interface may notify the person 1401 of sensed data (e.g., glucose level) and/or insulin delivery data (e.g., rates of historic, current, or future insulin delivery) and may present alerts to the person 1401. In some embodiments, a user interface may receive inputs from the person 1401, which may include, for example, a requested change in insulin delivery and/or a meal indication, among other things. The descriptions and embodiments above regarding user interfaces are merely provided as examples, and other types and other uses of user interfaces are contemplated to be within the scope of the present disclosure.

The following describes communications between the devices 1402-1406 and cooperation between the devices 1402-1406 with respect to insulin delivery. As depicted in FIG. 14, and as mentioned above, the devices 1402-1406 may communicate with each other via communication links 1412-1416. In some embodiments, the computing device 1406 may control operation of the delivery device 1402 and/or the monitoring device 1404. For example, the computing device 1406 may generate one or more signals (e.g., a command signal) that cause the delivery device 1402 to deliver insulin to the person 1401, e.g., as a basal dosage and/or a bolus dosage. In some embodiments, the computing device 1406 may receive data associated with insulin delivery (e.g., insulin delivery data) from the delivery device 1402 and/or receive sensed data (e.g., glucose levels) from the monitoring device 1404 and may perform computations based on the insulin delivery data, the sensed data, and/or other data to control the delivery device 1402. Insulin delivery data may include, but is not limited to, a type of insulin being delivered, historical insulin delivery rates and/or amounts, current insulin delivery rate and/or amount, and/or user input affecting insulin delivery. As persons skilled in the art will understand, in a closed-loop operating mode, computing device 1406 may communicate dosage commands to the delivery device 1402 based on a difference between a current glucose level in the body of the person 1401 (e.g., received from the monitoring device 1404) and a target glucose level (e.g., determined by the computing device 1406). The dosage commands may indicate an amount of insulin to be delivered and/or a rate of insulin delivery and may regulate the current glucose level toward the target glucose level. Examples of closed-loop operations for insulin infusion systems are described in United States Patent Nos.: 6,088,608, 6,119,028, 6,589,229, 6,740,072, 6,827,702, 7,323,142, and 7,402,153, and in United States Patent Application Publication Nos.: 2014/0066887 and 2014/0066889. The entire contents of each of the foregoing patents and publications are hereby incorporated by reference herein.

The remote or cloud computing system 1408 may be a proprietary remote/cloud computing system or a commercial cloud computing system including one or more server computing devices. The remote/cloud computing system 1408 may provide additional computing resources on-demand as needed when the computing resources of a client computing device (e.g., the computing device 1406) are not sufficient. The computing device 1406 and the remote/cloud computing system 108 may communicate with each other through a communication link 1418, which may traverse one or more communication networks (not shown). The communication networks may include, without limitation, an Ethernet network, Wi-Fi network, a cellular network, a satellite network, an intranet, an extranet, the Internet, and/or the Internet backbone, among other types of networks. Persons skilled in the art will recognize implementations for the remote/cloud computing system 1408 and how to interface with such systems through various types of networks. For example, the remote/cloud computing system 1408 may include an array of processing circuitry (defined above) and may execute machine-readable instructions. Such implementations, interfaces, and networks are contemplated to be within the scope of the present disclosure.

In some embodiments, therapy may be effected based on communicating a therapy determination toward a therapy delivery device. Using FIG. 14 as an example, a therapy determination (e.g., an insulin amount or adjusted insulin amount) may be communicated from the remote/cloud computing system 1408 to the delivery device 1402 via the computing device 1406. Alternatively, as discussed in connection with the example system of FIG. 1, a therapy determination may be communicated to a therapy delivery device from a monitoring device, either directly or through an intermediary such as the computing device 1406 in FIG. 14.

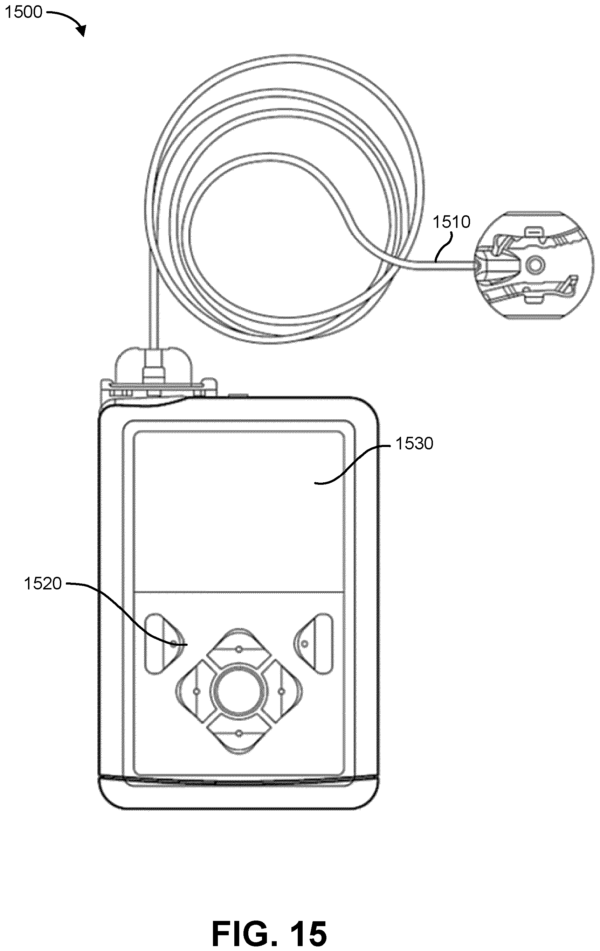

**FIG. 15** depicts an example insulin delivery device 1500, which may implement a therapy delivery device in any of the systems described above (e.g., delivery device 120 or delivery device 1402). In an insulin delivery device such as the device 1500, insulin delivery may be performed based on internal communication between a central computing module (e.g., a microcontroller for device 1500 as a whole) and an insulin delivery module (e.g., including a microcontroller, a motor, and a pump). For instance, insulin delivery may be caused by the central computing module communicating a delivery command in the form of an electrical signal that travels via a communication fabric to the insulin delivery module. The central computing module may also be configured to communicate (e.g., via a transceiver) with a computing device (e.g., 1406, FIG. 14) communicatively coupled to a remote or cloud computing system (e.g., 1408, FIG. 14). The delivery device 1500 may communicate various event data toward the remote or cloud computing system, which may communicate insulin delivery determinations toward the delivery device 1500, in accordance with the techniques described herein.

The insulin delivery device 1500 can provide fast-acting insulin through a small tube 1510 configured for fluidic connection with a cannula inserted subcutaneously. The device 1500 can deliver two types of dosages-a basal dosage, which can be delivered periodically (e.g., every five minutes) in tiny amounts throughout the day and night, and a bolus dosage to cover an increase in blood glucose from meals and/or to correct high blood glucose levels. The depicted insulin delivery device 1500 includes a user interface having button elements 1520 that can be manipulated to administer a bolus of insulin, to change therapy settings, to change user preferences, to select display features, and the like. The insulin delivery device 1500 also includes a display device 1530 that can be used to present various types of information or data to the user. In accordance with aspects of the present disclosure, a user of the insulin delivery device 1500 may use the button elements 1520 to input certain event data (e.g., event type, event start time, event details, etc.), and the user inputs can be confirmed using the display device 1530. The insulin delivery device 1500 of FIG. 12 is merely provided by way of example, and other types of insulin delivery devices and other techniques different from those described above are contemplated to be within the scope of the present disclosure.

The embodiments disclosed herein are examples of the disclosure and may be embodied in various forms. For instance, although certain embodiments herein are described as separate embodiments, each of the embodiments herein may be combined with one or more of the other embodiments herein. Various embodiments may omit, substitute, or add various procedures or components as appropriate. For instance, in alternative configurations, the methods described may be performed in an order different from that described, and/or various stages may be added, omitted, and/or combined. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to like elements throughout the description of the figures.

Any of the herein described techniques, operations, methods, programs, algorithms, or codes may be converted to, or expressed in, a programming language or computer program embodied on a computer, processor, or machine-readable medium. The terms "programming language" and "computer program," as used herein, each include any language used to specify instructions to a computer or processor, and include (but is not limited to) the following languages and their derivatives: Assembler, Basic, Batch files, BCPL, C, C+, C++, Delphi, Fortran, Java, JavaScript, machine code, operating system command languages, Pascal, Perl, Programming Language One (PL/1), Python, scripting languages, Visual Basic, metalanguages which themselves specify programs, and all first, second, third, fourth, fifth, or further generation computer languages. Also included are database and other data schemas, and any other metalanguages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

Terms, "and" and "or" as used herein, may include a variety of meanings that are also expected to depend at least in part upon the context in which such terms are used. Typically, "or" if used to associate a list, such as A, B, or C, is intended to mean A, B, and C, here used in the inclusive sense, as well as A, B, or C, here used in the exclusive sense. In addition, the term "one or more" as used herein may be used to describe any feature, structure, or characteristic in the singular or may be used to describe some combination of features, structures, or characteristics. However, it should be noted that this is merely an illustrative example and claimed subject matter is not limited to this example. Furthermore, the term "at least one of" if used to associate a list, such as A, B, or C, can be interpreted to mean any combination of A, B, and/or C, such as A, AB, AC, BC, AA, ABC, AAB, AABBCCC, etc.

It should be understood that the foregoing description is only illustrative of the present disclosure. To the extent consistent, any or all of the aspects detailed herein may be used in conjunction with any or all of the other aspects detailed herein. Various alternatives and modifications can be devised by those skilled in the art without departing from the disclosure. Accordingly, the present disclosure is intended to embrace all such alternatives, modifications, and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure.

While several embodiments of the disclosure have been depicted in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

In view of this description embodiments may include different combinations of features. Implementation examples are described in the following numbered clauses:

Clause 1. A system comprising: one or more processors; and one or more processor-readable media storing instructions which, when executed by the one or more processors, cause performance of: obtaining, based on glucose measurements performed on a person, a glucose value and a glucose rate of change (ROC); calculating an initial insulin amount to deliver to the person based on the glucose value; determining that the glucose value exceeds a glucose threshold, and that the glucose ROC exceeds an ROC threshold; obtaining an adjustment value in response to the determination, wherein: the adjustment value represents an adjustment to be applied to the initial insulin amount when the glucose threshold and the ROC threshold are exceeded, and the adjustment value was determined based on earlier glucose measurements performed on the person; and adjusting the initial insulin amount according to the adjustment value.

Clause 2. The system of clause 1, wherein when executed by the one or more processors, the instructions cause the system to adjust the initial insulin amount through at least one of:

communicating the adjustment value or a corresponding adjusted insulin amount to an insulin delivery device; or presenting, through an output device, the adjustment value or corresponding adjusted insulin amount to a user.

**Clause 3.** The system of clause 1 or 2, wherein the adjustment value is a scaling factor multiplied with the initial insulin amount to calculate an adjusted insulin amount.

**Clause 4.** The system of any of clauses 1-3, wherein the glucose threshold is a value specific to the person.

**Clause 5.** The system of any of clauses 1-4, wherein the ROC threshold is a value specific to the person.

**Clause 6.** The system of any of clauses 1-5, wherein the glucose threshold, the ROC threshold, and the adjustment value were determined through a simulation of the person's glucose level under different insulin dosage scenarios, using a physiological model derived from the earlier glucose measurements.

**Clause 7.** The system of clause 6, wherein: the simulation involves identifying different combinations of glucose threshold, ROC threshold, and adjustment value that are associated with increased time spent within a target glucose range relative to delivery of unadjusted insulin doses, and the glucose threshold, the ROC threshold, and the adjustment value are based on the identified combinations.

**Clause 8.** The system of any of clauses 1-7, wherein the adjustment value is stored in a memory accessible to the one or more processors and in association with the glucose threshold and the ROC threshold, and wherein obtaining the adjustment value comprises retrieving the adjustment value from the memory.

**Clause 9.** The system of any of clauses 1-8, wherein: the glucose threshold is at least 100 mg/dL and is exceeded when the glucose value rises above the glucose threshold; and the ROC threshold is a positive value of 0.25 mg/dL per minute or higher and is exceeded when the glucose ROC rises above the ROC threshold.

**Clause 10.** The system of any of clauses 1-8, wherein: the glucose threshold is at most 150 mg/dL and is exceeded when the glucose value falls below the glucose threshold; and the ROC threshold is a negative value of -0.25 mg/dL per minute or lower and is exceeded when the glucose ROC falls below the ROC threshold.

**Clause 11.** A processor-implemented method comprising: obtaining, based on glucose measurements performed on a person, a glucose value and a glucose rate of change (ROC); calculating, by one or more processors of a computing device, an initial insulin amount to deliver to the person based on the glucose value; determining, by the one or more processors, that the glucose value exceeds a glucose threshold, and that the glucose ROC exceeds an ROC threshold; obtaining an adjustment value in response to the determination, wherein: the adjustment value represents an adjustment to be applied to the initial insulin amount when the glucose threshold and the ROC threshold are exceeded, and the adjustment value was determined based on earlier glucose measurements performed on the person; and adjusting, by the computing device, the initial insulin amount according to the adjustment value.

**Clause 12.** The processor-implemented method of clause 11, wherein adjusting the initial insulin amount comprises at least one of: communicating the adjustment value or a corresponding adjusted insulin amount to an insulin delivery device; or presenting, through an output device, the adjustment value or corresponding adjusted insulin amount to a user.

**Clause 13.** The processor-implemented method of clause 11 or 12, wherein the adjustment value is a scaling factor multiplied with the initial insulin amount to calculate an adjusted insulin amount.

**Clause 14.** The processor-implemented method of any of clauses 11-13, wherein the glucose threshold, the ROC threshold, and the adjustment value were determined through a simulation of the person's glucose level under different insulin dosage scenarios, using a physiological model derived from the earlier glucose measurements.

**Clause 15.** The processor-implemented method of clause 14, wherein: the simulation involves identifying different combinations of glucose threshold, ROC threshold, and adjustment value that are associated with increased time spent within a target glucose range relative to delivery of unadjusted insulin doses, and the glucose threshold, the ROC threshold, and the adjustment value are based on the identified combinations.

**Clause 16.** The processor-implemented method of any of clauses 11-15, wherein the adjustment value is stored in a memory accessible to the one or more processors and in association with the glucose threshold and the ROC threshold, and wherein obtaining the adjustment value comprises retrieving the adjustment value from the memory.

**Clause 17.** The processor-implemented method of any of clauses 11-16, wherein: the glucose threshold is at least 100 mg/dL and is exceeded when the glucose value rises above the glucose threshold; and the ROC threshold is a positive value of 0.25 mg/dL per minute or higher and is exceeded when the glucose ROC rises above the ROC threshold.

**Clause 18.** The processor-implemented method of any of clauses 11-16, wherein: the glucose threshold is at most 150 mg/dL and is exceeded when the glucose value falls below the glucose threshold; and the ROC threshold is a negative value of -0.25 mg/dL per minute or lower and is exceeded when the glucose ROC falls below the ROC threshold.

**Clause 19.** One or more processor-readable media storing instructions which, when executed by one or more processors, cause performance of: obtaining, based on glucose measurements performed on a person, a glucose value and a glucose rate of change (ROC) value; calculating an initial insulin amount to deliver to the person based on the glucose value; determining that the glucose value exceeds a glucose threshold, and that the glucose ROC exceeds an ROC threshold; obtaining an adjustment value in response to the determination, wherein: the adjustment value represents an adjustment to be applied to the initial insulin amount when the glucose threshold and the ROC threshold are exceeded, and the adjustment value was determined based on earlier glucose measurements performed on the person; and adjusting the initial insulin amount according to the adjustment value.

**Clause 20.** The processor-readable media of clause 19, wherein: the glucose threshold, the ROC threshold, and the adjustment value were determined through a simulation of the person's glucose level under different insulin dosage scenarios, using a physiological model derived from the earlier glucose measurements, and the simulation involves identifying different combinations of glucose threshold, ROC threshold, and adjustment value that are associated with increased time spent within a target glucose range relative to delivery of unadjusted insulin doses.

Further disclosed herein is the subject-matter of the following items:

1. A system comprising:

one or more processors; and
one or more processor-readable media storing instructions which, when executed by the one or more processors, cause performance of:

obtaining, based on glucose measurements performed on a person, a glucose value and a glucose rate of change (ROC);
calculating an initial insulin amount to deliver to the person based on the glucose value;
determining that the glucose value exceeds a glucose threshold, and that the glucose ROC exceeds an ROC threshold;
obtaining an adjustment value in response to the determination, wherein:

the adjustment value represents an adjustment to be applied to the initial insulin amount when the glucose threshold and the ROC threshold are exceeded, and
the adjustment value was determined based on earlier glucose measurements performed on the person; and

adjusting the initial insulin amount according to the adjustment value.

2. The system of item 1, wherein when executed by the one or more processors, the instructions cause the system to adjust the initial insulin amount through at least one of:

communicating the adjustment value or a corresponding adjusted insulin amount to an insulin delivery device; or
presenting, through an output device, the adjustment value or corresponding adjusted insulin amount to a user.

3. The system of item 1 or 2, wherein the adjustment value is a scaling factor multiplied with the initial insulin amount to calculate an adjusted insulin amount.

4. The system of item 1 or of any of items 1 to 3, wherein the glucose threshold is a value specific to the person.

5. The system of item 1 or of any of items 1 to 4, wherein the ROC threshold is a value specific to the person.

6. The system of item 1 or of any of items 1 to 5, wherein the glucose threshold, the ROC threshold, and the adjustment value were determined through a simulation of the person's glucose level under different insulin dosage scenarios, using a physiological model derived from the earlier glucose measurements.

7. The system of item 6 or of any of items 1 to 6, wherein:

the simulation involves identifying different combinations of glucose threshold, ROC threshold, and adjustment value that are associated with increased time spent within a target glucose range relative to delivery of unadjusted insulin doses, and
the glucose threshold, the ROC threshold, and the adjustment value are based on the identified combinations.

8. The system of item 1 or of any of items 1 to 7, wherein the adjustment value is stored in a memory accessible to the one or more processors and in association with the glucose threshold and the ROC threshold, and wherein obtaining the adjustment value comprises retrieving the adjustment value from the memory.

9. The system of item 1 or of any of items 1 to 8, wherein:

the glucose threshold is at least 100 mg/dL and is exceeded when the glucose value rises above the glucose threshold; and
the ROC threshold is a positive value of 0.25 mg/dL per minute or higher and is exceeded when the glucose ROC rises above the ROC threshold.

10. The system of item 1 or of any of items 1 to 9, wherein:

the glucose threshold is at most 150 mg/dL and is exceeded when the glucose value falls below the glucose threshold; and
the ROC threshold is a negative value of -0.25 mg/dL per minute or lower and is exceeded when the glucose ROC falls below the ROC threshold.

11. A processor-implemented method comprising:

obtaining, based on glucose measurements performed on a person, a glucose value and a glucose rate of change (ROC);
calculating, by one or more processors of a computing device, an initial insulin amount to deliver to the person based on the glucose value;
determining, by the one or more processors, that the glucose value exceeds a glucose threshold, and that the glucose ROC exceeds an ROC threshold;
obtaining an adjustment value in response to the determination, wherein:

the adjustment value represents an adjustment to be applied to the initial insulin amount when the glucose threshold and the ROC threshold are exceeded, and
the adjustment value was determined based on earlier glucose measurements performed on the person; and

adjusting, by the computing device, the initial insulin amount according to the adjustment value.

12. The processor-implemented method of item 11, wherein adjusting the initial insulin amount comprises at least one of:

communicating the adjustment value or a corresponding adjusted insulin amount to an insulin delivery device; or
presenting, through an output device, the adjustment value or corresponding adjusted insulin amount to a user.

13. The processor-implemented method of item 11 or 12, wherein the adjustment value is a scaling factor multiplied with the initial insulin amount to calculate an adjusted insulin amount.

14. The processor-implemented method of item 11 or of any of items 11 to 13, wherein the glucose threshold, the ROC threshold, and the adjustment value were determined through a simulation of the person's glucose level under different insulin dosage scenarios, using a physiological model derived from the earlier glucose measurements.

15. The processor-implemented method of item 14 or of any of items 11 to 14, wherein:

the simulation involves identifying different combinations of glucose threshold, ROC threshold, and adjustment value that are associated with increased time spent within a target glucose range relative to delivery of unadjusted insulin doses, and
the glucose threshold, the ROC threshold, and the adjustment value are based on the identified combinations.

16. The processor-implemented method of item 11 or of any of items 11 to 15, wherein the adjustment value is stored in a memory accessible to the one or more processors and in association with the glucose threshold and the ROC threshold, and wherein obtaining the adjustment value comprises retrieving the adjustment value from the memory.

17. The processor-implemented method of item 11 or of any of items 11 to 16, wherein:

the glucose threshold is at least 100 mg/dL and is exceeded when the glucose value rises above the glucose threshold; and

the ROC threshold is a positive value of 0.25 mg/dL per minute or higher and is exceeded when the glucose ROC rises above the ROC threshold.

18. The processor-implemented method of item 11 or of any of items 11 to 17, wherein:

the glucose threshold is at most 150 mg/dL and is exceeded when the glucose value falls below the glucose threshold; and
the ROC threshold is a negative value of -0.25 mg/dL per minute or lower and is exceeded when the glucose ROC falls below the ROC threshold.

19. One or more processor-readable media storing instructions which, when executed by one or more processors, cause performance of:

obtaining, based on glucose measurements performed on a person, a glucose value and a glucose rate of change (ROC) value;
calculating an initial insulin amount to deliver to the person based on the glucose value;
determining that the glucose value exceeds a glucose threshold, and that the glucose ROC exceeds an ROC threshold;
obtaining an adjustment value in response to the determination, wherein:

the adjustment value represents an adjustment to be applied to the initial insulin amount when the glucose threshold and the ROC threshold are exceeded, and
the adjustment value was determined based on earlier glucose measurements performed on the person; and

adjusting the initial insulin amount according to the adjustment value.

20. The processor-readable media of item 19, wherein:

the glucose threshold, the ROC threshold, and the adjustment value were determined through a simulation of the person's glucose level under different insulin dosage scenarios, using a physiological model derived from the earlier glucose measurements, and
the simulation involves identifying different combinations of glucose threshold, ROC threshold, and adjustment value that are associated with increased time spent within a target glucose range relative to delivery of unadjusted insulin doses.

## Claims

1. A system comprising:

one or more processors; and
one or more processor-readable media storing instructions which, when executed by the one or more processors, cause performance of:

obtaining, based on glucose measurements performed on a person, a glucose value and a glucose rate of change (ROC);
calculating an initial insulin amount to deliver to the person based on the glucose value;
determining that the glucose value exceeds a glucose threshold, and that the glucose ROC exceeds an ROC threshold;
obtaining an adjustment value in response to the determination, wherein:

the adjustment value represents an adjustment to be applied to the initial insulin amount when the glucose threshold and the ROC threshold are exceeded, and
the adjustment value was determined based on earlier glucose measurements performed on the person; and

adjusting the initial insulin amount according to the adjustment value.

2. The system of claim 1, wherein when executed by the one or more processors, the instructions cause the system to adjust the initial insulin amount through at least one of:

> communicating the adjustment value or a corresponding adjusted insulin amount to an insulin delivery device; or
> presenting, through an output device, the adjustment value or corresponding adjusted insulin amount to a user.

3. The system of claim 1 or 2, wherein the adjustment value is a scaling factor multiplied with the initial insulin amount to calculate an adjusted insulin amount, and/or

> wherein the glucose threshold is a value specific to the person, and/or
> wherein the ROC threshold is a value specific to the person.

4. The system of any of claims 1 to 3, wherein the glucose threshold, the ROC threshold, and the adjustment value were determined through a simulation of the person's glucose level under different insulin dosage scenarios, using a physiological model derived from the earlier glucose measurements, particularly wherein:

> the simulation involves identifying different combinations of glucose threshold, ROC threshold, and adjustment value that are associated with increased time spent within a target glucose range relative to delivery of unadjusted insulin doses, and
> the glucose threshold, the ROC threshold, and the adjustment value are based on the identified combinations.

5. The system of any of claims 1 to 4, wherein the adjustment value is stored in a memory accessible to the one or more processors and in association with the glucose threshold and the ROC threshold, and wherein obtaining the adjustment value comprises retrieving the adjustment value from the memory.

6. The system of any of claims 1 to 5, wherein:

> the glucose threshold is at least 100 mg/dL and is exceeded when the glucose value rises above the glucose threshold; and
> the ROC threshold is a positive value of 0.25 mg/dL per minute or higher and is exceeded when the glucose ROC rises above the ROC threshold.

7. The system of any of claims 1 to 6, wherein:

> the glucose threshold is at most 150 mg/dL and is exceeded when the glucose value falls below the glucose threshold; and
> the ROC threshold is a negative value of -0.25 mg/dL per minute or lower and is exceeded when the glucose ROC falls below the ROC threshold.

8. A processor-implemented method comprising:

> obtaining, based on glucose measurements performed on a person, a glucose value and a glucose rate of change (ROC);
> calculating, by one or more processors of a computing device, an initial insulin amount to deliver to the person based on the glucose value;
> determining, by the one or more processors, that the glucose value exceeds a glucose threshold, and that the glucose ROC exceeds an ROC threshold;
> obtaining an adjustment value in response to the determination, wherein:
>
>> the adjustment value represents an adjustment to be applied to the initial insulin amount when the glucose threshold and the ROC threshold are exceeded, and
>> the adjustment value was determined based on earlier glucose measurements performed on the person; and
>
> adjusting, by the computing device, the initial insulin amount according to the adjustment value.

9. The processor-implemented method of claim 8, wherein adjusting the initial insulin amount comprises at least one of:

communicating the adjustment value or a corresponding adjusted insulin amount to an insulin delivery device; or presenting, through an output device, the adjustment value or corresponding adjusted insulin amount to a user.

10. The processor-implemented method of claim 8 or 9, wherein the adjustment value is a scaling factor multiplied with the initial insulin amount to calculate an adjusted insulin amount.

11. The processor-implemented method of any of claims 8 to 10, wherein the glucose threshold, the ROC threshold, and the adjustment value were determined through a simulation of the person's glucose level under different insulin dosage scenarios, using a physiological model derived from the earlier glucose measurements, particularly wherein:

the simulation involves identifying different combinations of glucose threshold, ROC threshold, and adjustment value that are associated with increased time spent within a target glucose range relative to delivery of unadjusted insulin doses, and

the glucose threshold, the ROC threshold, and the adjustment value are based on the identified combinations.

12. The processor-implemented method of any of claims 8 to 11, wherein the adjustment value is stored in a memory accessible to the one or more processors and in association with the glucose threshold and the ROC threshold, and wherein obtaining the adjustment value comprises retrieving the adjustment value from the memory.

13. The processor-implemented method of any of claims 8 to 12, wherein:

the glucose threshold is at least 100 mg/dL and is exceeded when the glucose value rises above the glucose threshold; and

the ROC threshold is a positive value of 0.25 mg/dL per minute or higher and is exceeded when the glucose ROC rises above the ROC threshold.

14. The processor-implemented method of any of claims 8 to 13, wherein:

the glucose threshold is at most 150 mg/dL and is exceeded when the glucose value falls below the glucose threshold; and

the ROC threshold is a negative value of -0.25 mg/dL per minute or lower and is exceeded when the glucose ROC falls below the ROC threshold.

15. One or more processor-readable media storing instructions which, when executed by one or more processors, cause performance of:

obtaining, based on glucose measurements performed on a person, a glucose value and a glucose rate of change (ROC) value;

calculating an initial insulin amount to deliver to the person based on the glucose value;

determining that the glucose value exceeds a glucose threshold, and that the glucose ROC exceeds an ROC threshold;

obtaining an adjustment value in response to the determination, wherein:

the adjustment value represents an adjustment to be applied to the initial insulin amount when the glucose threshold and the ROC threshold are exceeded, and

the adjustment value was determined based on earlier glucose measurements performed on the person; and

adjusting the initial insulin amount according to the adjustment value, particularly

wherein:

the glucose threshold, the ROC threshold, and the adjustment value were determined through a simulation of the person's glucose level under different insulin dosage scenarios, using a physiological model derived from the earlier glucose measurements, and

the simulation involves identifying different combinations of glucose threshold, ROC threshold, and adjustment value that are associated with increased time spent within a target glucose range relative to delivery of unadjusted insulin doses.

FIG. 1

**FIG. 2**

EP 4 528 744 A1

FIG. 4

FIG. 3

FIG. 5A

FIG. 5B

FIG. 6

EP 4 528 744 A1

FIG. 7

EP 4 528 744 A1

**FIG. 8**

EP 4 528 744 A1

FIG. 9A

FIG. 9B

EP 4 528 744 A1

FIG. 10A

FIG. 10B

EP 4 528 744 A1

1100

1102 — Obtain a glucose value and a glucose ROC based on glucose measurements performed on a person

1104 — Calculate a initial insulin amount to deliver to the person based on the glucose value

1106 — Determine that the glucose value exceeds a glucose threshold and that the glucose ROC exceeds an ROC threshold

1108 — Obtain an adjustment value in response to the determination in 1106, where the adjustment value represents an adjustment to be applied to the initial insulin amount when the glucose threshold and the ROC threshold are exceeded, and
where the adjustment value was determined based on earlier glucose measurements performed on the person

1110 — Adjust the initial insulin amount according to the adjustment value

# FIG.11

1200

1202 — Obtain glucose measurements and information on glucose-related events (e.g., meals, insulin doses) that occurred during time periods covered by the glucose measurements

1204 — Derive a physiological model from the glucose measurements and event information from 1202 to generate a person-specific model

1206 — Generate candidate parameter sets using different possible combinations of glucose thresholds, ROC thresholds, and adjustment values

1208 — Apply the person-specific model to simulate glucose level under different dosage scenarios, using the candidate parameter sets as input to the person-specific model

1210 — Eliminate candidate parameter sets not associated with increased time-in-range or associated with increased time-below-range

1212 — Determine values for an adjustment parameter set based on the remaining candidate parameter sets

# FIG.12

1300

1302 — Obtain, based on glucose measurements performed on a person, a glucose value and a glucose ROC

1304 — Calculate a initial insulin amount to deliver to the person based on the glucose value

1306 — Determine an adjusted insulin amount

1308 — Simulate future glucose levels using a physiological model specific to the person, where the future glucose levels represent a predicted result of delivering the adjusted insulin amount in conjunction with the glucose value and the glucose ROC obtained in 1302

1310 — Determine that one or more of the future glucose levels exceed a threshold associated with a target glucose range and/or meet other criteria

1312 — In response to the determination in 1310, cause the initial insulin amount to be delivered instead of the adjusted insulin amount

# FIG.13

1400

1401

Monitoring
Device
1404

1412

Delivery
Device
1402

1414

Computing
Device
1406

Remote
Computing
System
1408

1418

1416

FIG. 14

**FIG. 15**

Europäisches
Patentamt

European
Patent Office

Office européen
des brevets

# EUROPEAN SEARCH REPORT

**Application Number**

EP 24 19 9929

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/235714 A1 (BETA BIONICS INC [US]) 10 November 2022 (2022-11-10) * paragraph [0425] - paragraph [0512]; figures 19-22 * | 1-15 | INV. G16H20/17 G16H50/50 |
| X | US 2022/375566 A1 (KIRCHER JR ROBERT C [US] ET AL) 24 November 2022 (2022-11-24) * paragraph [0109]; figure 4 * * paragraph [0110] - paragraph [0143]; figures 5A-5C * * paragraph [0175]; figure 6 * | 1-15 | |
| X | US 2013/338629 A1 (AGRAWAL PRATIK [US] ET AL) 19 December 2013 (2013-12-19) * paragraph [0179] * | 1-15 | |

| TECHNICAL FIELDS SEARCHED (IPC) |
|---|
| G16H |

The present search report has been drawn up for all claims

| Place of search | Date of completion of the search | Examiner |
|---|---|---|
| Munich | 7 February 2025 | Eichenauer, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 9929

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2025

| Patent document cited in search report | Publication date | Patent family member(s) | | Publication date |
|---|---|---|---|---|
| WO 2022235714 A1 | 10-11-2022 | NONE | | |
| US 2022375566 A1 | 24-11-2022 | US | 2010292634 A1 | 18-11-2010 |
| | | US | 2014031786 A1 | 30-01-2014 |
| | | US | 2016022904 A1 | 28-01-2016 |
| | | US | 2017258997 A1 | 14-09-2017 |
| | | US | 2019125968 A1 | 02-05-2019 |
| | | US | 2021016001 A1 | 21-01-2021 |
| | | US | 2022375566 A1 | 24-11-2022 |
| | | WO | 2007149533 A2 | 27-12-2007 |
| US 2013338629 A1 | 19-12-2013 | CA | 2874746 A1 | 12-12-2013 |
| | | CN | 104520862 A | 15-04-2015 |
| | | EP | 2859480 A1 | 15-04-2015 |
| | | US | 2013338629 A1 | 19-12-2013 |
| | | US | 2013338630 A1 | 19-12-2013 |
| | | US | 2013345663 A1 | 26-12-2013 |
| | | WO | 2013184896 A1 | 12-12-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63583095
- US 4562751 A
- US 4685903 A
- US 5080653 A
- US 5505709 A
- US 5097122 A
- US 6485465 B
- US 6554798 B
- US 6558320 B
- US 6558351 B
- US 6641533 B
- US 6659980 B
- US 6752787 B
- US 6817990 B
- US 6932584 B
- US 7621893 B
- US 6088608 A
- US 6119028 A
- US 6589229 B
- US 6740072 B
- US 6827702 B
- US 7323142 B
- US 7402153 B
- US 20140066887
- US 20140066889